# FASCICULE DE BREVET EUROPEEN

(11) **EP 0 792 369 B1**
(45) Date de publication et mention de la délivrance du brevet: **05.04.2000**
(21) Numéro de dépôt: 95940324.7
(22) Date de dépôt: 16.11.1995
(51) Int. Cl.: C12N 15/85, C12N 15/54, C12N 15/55, A61K 48/00, A61K 38/43

(54) **GENES SUICIDE ET ASSOCIATIONS D'ANALOGUES DE NUCLEOBASES ET NUCLEOSIDES PYRIMIDIQUES AVEC DES GENES SUICIDE EN VUE D'UNE THERAPIE GENIQUE**
SELBSTMORDGENE UND HYBRIDE AUS NUKLEOTID ODER PYRIMIDIN-NUKLEOSID-ANALOGEN MIT SELBSTMORDGENE FÜR DIE GENTHERAPIE
SUICIDE GENES AND COMBINATIONS OF PYRIMIDINE NUCLEOSIDE AND NUCLEOBASE ANALOGUES WITH SUICIDE GENES FOR GENE THERAPY

(30) Priorité: 17.11.1994 US 343923
(43) Date de publication de la demande: 03.09.1997
(73) Titulaire: CAYLA, F-31094 Toulouse Cédex (FR)
(72) Inventeur: TIRABY, Gérard, F-31400 Toulouse (FR); REYNES, Jean-Paul, F-31750 Escalquens (FR); TIRABY, Michèle, F-31400 Toulouse (FR); CAZAUX, Christophe, F-31830 Plaisance-du-Touch (FR); DROCOURT, Daniel, F-31650 Saint-Orens (FR)
(74) Mandataire: Boulinguiez, Didier
(86) Numéro de dépôt international: FR9501511
(87) Numéro de publication internationale: WO9616183

(56) Documents cités:
- EP-A- 0 402 108
- WO-A-93/01281
- PHARMACOLOGY AND THERAPY, vol. 63, no. 2, Août 1994 pages 199-207, C.A.MULLEN 'Metabolic suicide genes in gene therapy'
- JOURNAL OF CELLULAR BIOCHEMISTRY, no. supplement 21A, 10 Mars 1995 - 4 Avril 1995 page 433 G.TIRABY ET AL. 'Hybrid genes encoding both cytosine deaminase and uracil phosphoribosyltransferase as new suicide genes'

## Description

La présente invention concerne des thérapies moléculaires pour le traitement du cancer et des infections par le virus VIH dans lesquelles des cellules tumorales ou des cellules immunes sont transfectées de façon stable avec différents gènes introduits grâce à des systèmes de transfert hautement efficaces tels que des vecteurs rétroviraux ou adénoviraux.

Une des stratégies pour le traitement d'affections localisées est de rendre les cellules cibles sensibles à des agents chimiothérapiques normalement atoxiques, en utilisant des gènes dits "suicides" tels que le gène de la thymidine kinase du virus simplex de l'herpès (HSV-*tk*) ; le gène *tk* apparenté du virus Zoster de la varicelle ou le gène *gpt* de la xanthine/guanine phosphoribosyltransférase bactérienne. HSV-*tk*, par exemple, convertit le ganciclovir, analogue non toxique de la guanosine, en un composé phosphorylé qui agit en tant que terminateur de chaîne dans la synthèse de l'ADN, tuant sélectivement les cellules en division.

Le gène codA *d'Escherichia coli* qui encode la cytosine désaminase (désigné ci-après CDase) représente éventuellement un deuxième gène suicide pouvant être utilisé pour l'élimination sélective des cellules humaines indésirables. La cytosine désaminase est la première enzyme de l'unique voie métabolique par laquelle la cytosine exogène ou la cytosine endogène provenant du catabolisme des nucleotides pyrimidiques est utilisée par le biais d'une desamination hydrolytique pour donner de l'uracile et de l'ammoniaque. Des cytosines désaminases ont été trouvées chez des procaryotes et des eucaryotes inférieurs mais semblent être absentes chez des eucaryotes supérieurs, aussi bien chez les mammifères que chez les plantes. [(Koechlin et al., *Biochem Pharmacol.* 15 : 435-446 (1966) (Ross, C. *Plant Physiol.* 40 : 65-73 (1965)]. La CDase desamine également la fluorocytosine atoxique (désignée FC) en fluorouracile (désignée FU), composé très toxique quand il est efficacement converti en 5-fluoro-UMP. Les cellules dépourvues d'activité cytosine désaminase, soit suite à une mutation inactivante comme par exemple dans les mutants codA *d'Escherichia coli* et fcy1 de *Saccharomyces cerevisiae,* soit en raison de leur déficience naturelle pour cette enzyme comme le sont les cellules des mammifères et des plantes, sont résistantes à la 5-fluorocytosine [(Kilstrup et al. *J. Bacteriol.* 171 : 2124-2127 (1989) (Jund R. & Lacroute, F. *Journal of Bacteriology,* 102 : 607-615 (1970)]. Cette propriété est à l'origine de l'utilisation du gène *codA* d'*E.coli* comme gène suicide ou de sélection négative dans bon nombre d'expériences récemment publiées portant sur des cellules de mammifères et de plantes [(Huber et al., *Cancer Res.* 53 : 4619-4626 (1993) ; W0 93/01281 ; Mullen et al., *Cancer Res.* 1503-1506 (1994)] et dans lesquelles il a été montré que des cellules transformées ont acquis une activité cytosine désaminase et une sensibilité à la 5-fluorocytosine.

Dans la demande internationale de brevet W0 93/01281, Mullen et Blaese ont décrit un système de sélection négative comprenant un gène bactérien modifié de la cytosine désaminase possédant la capacité de produire un antimétabolite toxique à partir de la FC dans des cellules eucaryotes. De plus, Mullen et Blaese revendiquent une double sélection négative basée sur un gène modifié de la cytosine désaminase et sur le gène de la thymidine kinase herpétique dans une sélection impliquant la 5-fluorocytosine et le ganciclovir, un nucléoside acyclique analogue de la guanosine, dont les dérivés triphosphate entrent en compétition avec le dGTP. Cependant, il faut noter qu'une large majorité de cellules tumorales ne sont pas sensibles à cette méthode de thérapie.

La sensibilité différentielle à la FC des cellules parentales et des cellules transfectées par un gène de la cytosine désaminase varie beaucoup selon les lignées cellulaires. Presque aucune sensibilité à la fluorocytosine n'a été observée pour des clones murins de mélanome B16 transfectés par un vecteur contenant le gène codA et sélectionnés pour leur forte expression d'activité cytosine désaminase (comme illustré dans l'exemple 8 ci-après). La faible sensibilité à la fluorocytosine des cellules en culture exprimant une forte activité CDase, représente une situation rencontrée dans beaucoup de tumeurs, (J.D Harris, communication orale au colloque de Cold Spring Harbor Laboratory sur la thérapie génique), qui peut s'expliquer par au moins deux mécanismes possibles.

L'un est lié au transport de la cytosine. Toutes les nucléobases naturelles et leurs analogues fluorinés sont hydrophiles et ne diffusent que très lentement au travers de la membrane plasmique. Leur pénétration efficace est dépendante des protéines spécifiques du transport. Chez *E.coli,* le transport de la fluorocytosine est activé par une cytosine perméase spécifique codée par codB. Le rôle crucial d'un système actif de transport de la cytosine en déterminant la sensibilité de la fluorocytosine dans cette bactérie, a été démontré avec des mutants dans lequel le gène codB a été retiré. La situation normale apparaît être l'opposé chez les mammifères. Bien que les cellules de mammifères possèdent un système de transport de l'uracile, la cytosine n'est pas transportée de manière active dans diverses lignées cellulaires ou dans les erythrocytes humains, où la pénétration semble se faire exclusivement par diffusion passive [(Plagemann et al. *Biochim. Biophys. Acta.* 947 : 405-443 (1988)] .

L'autre mécanisme est basé sur l'existence et l'importance relative des voies anaboliques et cataboliques qui modulent la concentration en fluorouracile. Chez les microorganismes, l'uracile endogène est directement converti en UMP par l'uracile phosphoribosyltransférase (UPRTase) et peut également être converti en UMP et dUMP par l'action concertée de l'uridine phosphorylase et l'uridine kinase [(Munch-Petersen A. & Mygind B. *In. Metabolism of nucleotides, nucleosides and nucleobases in microorganisms.* (1983)] .

Cependant, cette voie en deux étapes ne fonctionne que si l'un des substrats de l'uridine phosphorylase, à savoir le ribose-1-phosphate ou le desoxyribose 1-phosphate, et l'uracile sont présents à de fortes concentrations intracellulaires. Chez les cellules de mammifères, l'uracile et le fluorouracile sont métabolisés en deux étapes en UMP et F-UMP via l'intermédiaire uridine [(Mulkins M.A.& Heidelberger, C. *Cancer Res.* 42 : 965-973 (1982) ; Schwartz et al. *Biochem Pharmacol.* 34 : 3585-3589 (1985)] en l'absence d'activité uracile phosphoribosyltransférase [(Kornberg A. & Baker T. *In DNA replication. Freeman, W.M and Company; New York,* 53-100 (1992)] . De plus, une proportion d'uracile et de fluorouracile est catabolisée respectivement en dihydrouracile atoxique et dihydrofluorouracile.

La toxicité de la FC pour des cellules exprimant un gène cytosine désaminase introduit dépend donc de la quantité de FU formé qui est converti en 5-fluoro-UMP et ensuite, via la voie *de novo* de la synthèse des pyrimidines, en 5-fluoro dUMP, un inhibiteur irréversible de la thymidylate synthétase et en conséquence de la synthèse de l'ADN par manque de dTTP [(Kornberg A. & Baker T. *In DNA replication. Freeman, W.M and Company; New York,* 53-100 (1992)] .

L'azidothymidine (AZT) utilisé pour le traitement de l'infection par le virus de l'immunodéficience humaine (SIDA) fait partie des médicaments qui sont métabolisés par la voie des nucléosides pyrimidiques.

L'AZT est un des premiers agents chimiothérapiques pour le traitement d'une infection VIH. L'AZT entre facilement dans les cellules où il est converti en nucléosides phosphate AZT-MP, AZT-DP, et AZT-TP par l'action séquentielle de la thymidine kinase, la thymidylate kinase et la nucleoside diphosphokinase. L'AZT est rapidement converti en AZT-MP par la thymidine kinase mais l'AZT-MP s'accumule en raison du fait qu'il est un mauvais substrat de la thymidylate kinase humaine. L'AZT-MP est trouvé de facon reproductible en concentration au moins 50 fois plus élevée que celle de l'AZT-TP dans des lignées cellulaires humaines [(Furman et al. *Proc. Natl. Acad. Sci. USA,* 83 : 8333-7 (1986)] . Bien que l'AZT-TP soit un substrat inefficace pour les ADN polymerases cellulaires, l'AZT-MP est incorporé dans l'ADN [(Copeland, J. *Biol. Chem.* 267 : 21459-64 (1992)].

La présente invention concerne un groupe de gènes d'origine bactérienne et fongique en entité seule codant pour une UPRTase ou fusionné avec des gènes bactériens et fongiques codant pour une CDase. Les gènes avec la seule activité l'UPRTase sensibilisent les cellules de mammifères au 5-FU alors que les gènes hybrides codant pour une enzyme à deux domaines ayant les activités CDase et UPRTase sensibilisent les cellules de mammifères au 5-FU et à la 5-FC et en particulier les cellules de mammifères qui ne sont pas sensibles à la FC lorsqu'elles expriment la seule CDase.

L'invention concerne également un second groupe de nouveaux gènes suicide qui sensibilisent des cellules de mammifères à l'azidothymidine (AZT).

L'invention concerne également des vecteurs eucaryotes contenant les deux groupes de gènes suicide ou l'un des groupes seul ainsi qu'une méthode de traitement in *situ* du cancer et de l'infection par le VIH par introduction des dits vecteurs chez les malades.

Cette invention s'applique à des situations où les cellules de mammifères transfectées par un gène d'une cytosine désaminase seul ne sont pas sensibles à la FC comme c'est le cas avec de nombreuses cellules tumorales.

La société demanderesse propose l'utilisation d'un gène modifié codant pour une protéine unique à double activité cytosine désaminase et UPRTase dans des cellules tumorales faiblement sensibles à la FC quand elles sont transfectées par un gène codant uniquement pour la cytosine désaminase. Le raisonnement est basé sur l'hypothèse que dans le cas de deux protéines de fusion, le FU est plus rapidement et plus efficacement converti en 5-fluoro-UMP. Cela devrait en retour générer une augmentation du flux de la FC entrant dans les cellules, particulièrement dans des cellules tumorales dépourvues d'un transport actif de FC. L'exemple 8 illustre l'augmentation considérable de l'effet toxique observé après traitement à la FC, dans des cellules murines de mélanome B16 transfectés, conformément à l'invention, avec un gène hybride *codA::upp* en comparaison avec des cellules B16 transfectées avec le gène *codA* seul. Ces nouveaux gènes suicide, résultant de la fusion traductionnelle entre un gène de la cytosine désaminase et un gène de l'UPRTase, doivent être utilisés de préférence dans toute situation où les cellules cibles doivent être éliminées ou lorsque la croissance des cellules cibles doit être contrôlée.

Il a été démontré que l'ordre de la fusion UPRTase::CDase induit peu ou pas de différence dans l'activité spécifique de l'enzyme comparée à l'enzyme de fusion CDase::UPRTase. Le gène hybride *codA::upp* d'*E.coli* semble être tout aussi efficace que le gène hybride *fcy1::fur1* de *S.cerevisiae.* Le gène *fcy1::upp,* plus petit, pourrait être préféré avec des transporteurs tels que des vecteurs rétroviraux, AAV ou adénoviraux dans lesquels l'espace pour insérer le gène est limité. Les gènes bifonctionnels codant pour les activités CDase et UPRTase et les gènes codant pour la seule activité UPRTase constituent le premier groupe de nouveaux gènes suicide de cette invention.

L'invention concerne également un second groupe de nouveaux gènes suicide qui sensibilisent des cellules de mammifères à l'azidothymidine (AZT) et/ou à des analogues de nucleosides pyrimidiques tels que la didesoxythymidine, la trifluoromethylthymidine, l'éthyldesoxyuridine, le bromovinyldesoxyuridine, le bromovinylarabinouracile.

Un gène codant pour une thymidylate kinase avec une bonne affinité pour l'AZT-MP devrait sensibiliser des cellules de mammifères à l'AZT en procurant plus d'AZT triphosphate pour l'incorporation dans l'ADN lors de son élongation causant ainsi l'arrêt de la chaîne et la mort cellulaire. Les enzymes responsables de l'anabolisme de l'AZT chez la bactérie *Escherichia coli,* qui est très sensible à cette molécule, sont de bons candidats qui remplissent cette exigence. Le gène de la thymidylate kinase *tmk* d'*E.coli* seul ou fusionné au gène de la thymidine kinase *tdk* d'*E.coli* ou au gène de la nucleoside diphosphokinase *ndk* d'*E.coli,* augmentent considérablement l'effet toxique de l'AZT sur des cellules murines de mélanome B16. Ces nouveaux gènes suicide peuvent être utilisés préférentiellement dans toute situation où les cellules cibles doivent être éliminées ou lorsque la croissance des cellules cibles doit être contrôlée.

Le gène *tk* du virus humain de l'herpès simplex code pour une enzyme à double activité thymidine kinase et thymidylate kinase. L'AZT est un mauvais substrat pour la TK-HSV1 même si l'enzyme peut phosphoryler une grande variété d'analogues de nucléosides pyrimidiques et puriques. Lors de ses travaux de recherche d'une thymidylate kinase d'origine virale, capable de phosphoryler l'AZT-MP, la société demanderesse a découvert que le gène *tk* du virus Epstein-Barr convertit de façon conséquente l'AZT-MP en AZT-DP. Le gène *TK-EBV* est donc un bon candidat comme gène suicide pour sensibiliser à l'AZT des cellules de mammifères résistantes à cette molécule. Un autre gène de grand intérêt est le gène *tmk* codant pour la thymidylate kinase d'*Escherichia coli.* Lors de la recherche d'un microorganisme naturellement sensible à une faible concentration d'AZT ajouté au milieu de croissance, indiquant un métabolisme efficace de l'AZT, la société demanderesse a trouvé que des souches dérivées d'*E.coli* K12 étaient les plus sensibles parmi plusieurs milliers de souches bactériennes et fongiques testées. Le gène *tmk* d'*E.coli* a été isolé par clonage et modifié, après que la séquence nucléosidique ait été déterminée, de telle sorte qu'une fusion génique active a pu être faite avec d'autres gènes métabolisant l'AZT susceptibles ainsi de potentialiser l'action du gène *tmk* seul . La fusion du gène *tmk* avec le gène codant pour la thymidylate kinase ou avec le gène *ndk* codant pour la nucléoside diphosphokinase, toutes deux provenant d'*E.coli,* sont deux exemples de nouveaux gènes hybrides capables de phosphoryler efficacement l'AZT. Le gène *tmk* et les gènes bifonctionnels codant pour la thymidylate kinase et une autre activité des voies de synthèse *de novo* ou de récupération des pyrimidines, constituent le second groupe de nouveaux gènes suicide de cette invention.

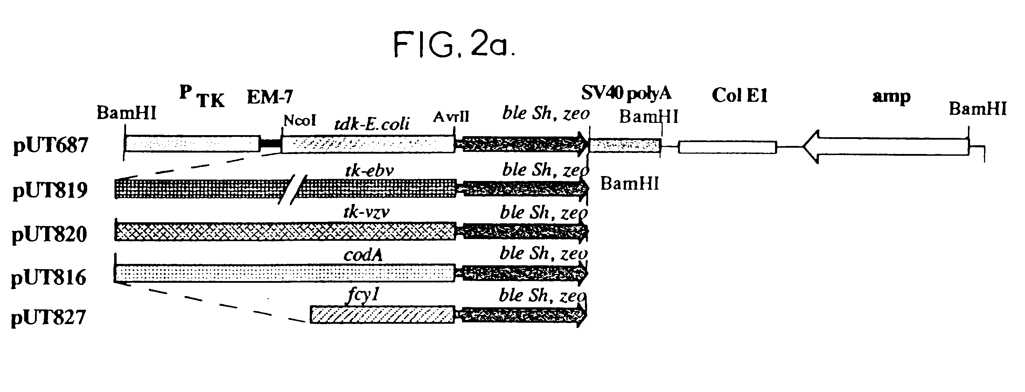

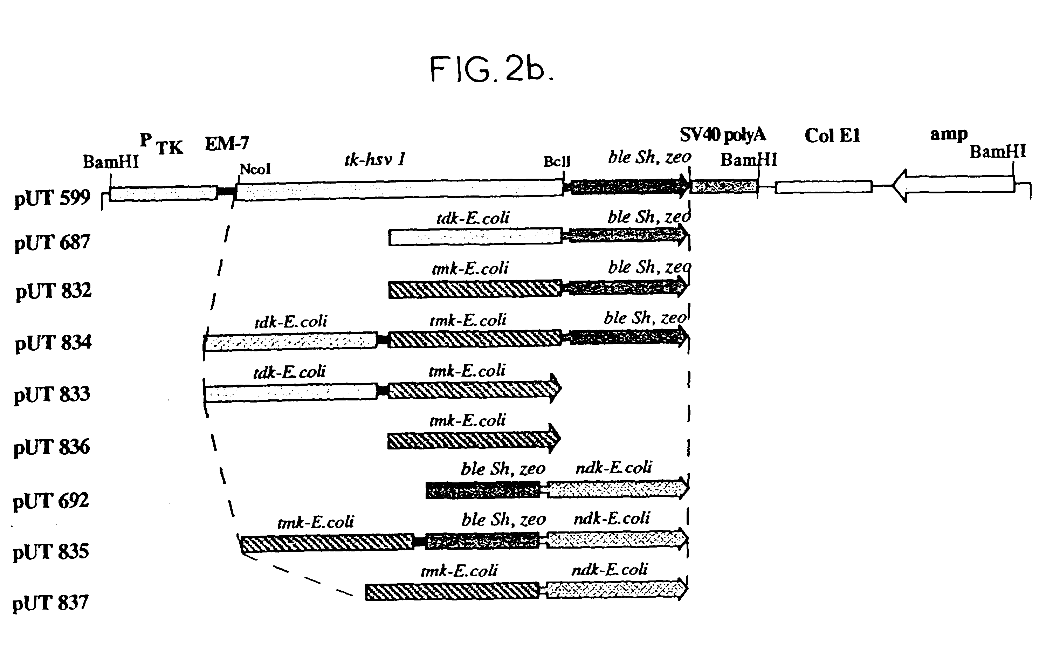

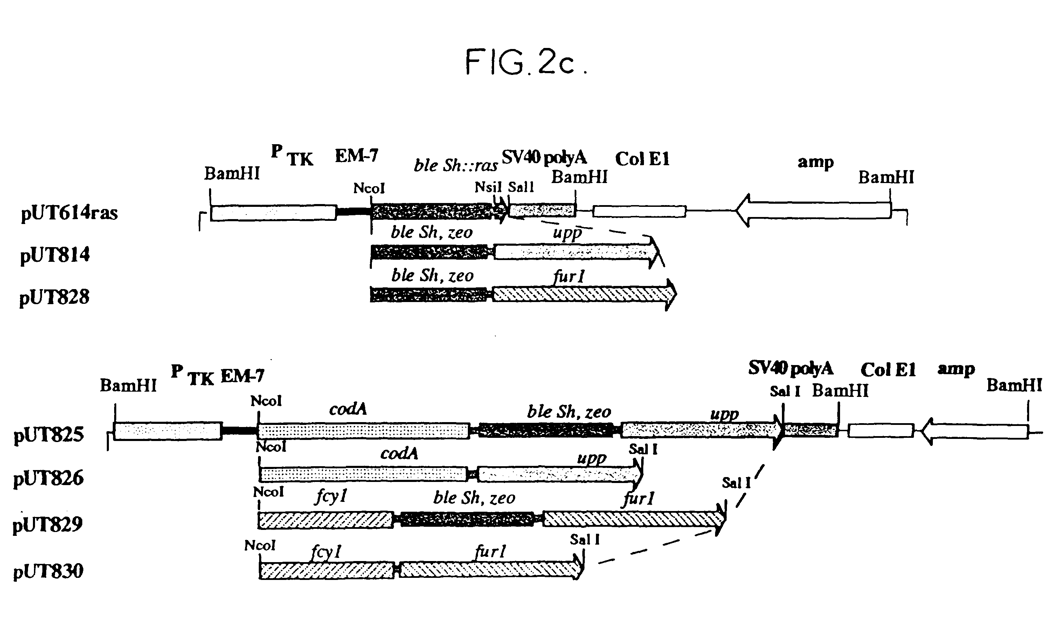

Selon une réalisation préférentielle de l'invention, qui rend les nouveaux gènes hybrides plus adaptés aux expériences de transfert d'ADN, les gènes monofonctionnels ou bifonctionnels des deux groupes peuvent être fusionnés dans un même cadre de lecture avec le petit gène bactérien *ble Sh,* rendant ainsi les cellules de mammifères résistantes à un antibiotique de la famille des phléomycines [(Armau et al., US Patents 5021344 et 5118620)]. Plusieurs constructions plasmidiques contenant le gène de sélection phléomycine/zeocine avec un phénotype négatif sont présentées dans les Figures 2a, 2b et 2c.

Il faut noter dans le cadre de cette invention que des gènes possédant des fonctions multiples ne sont pas des cas exceptionnels chez les eucaryotes supérieurs. Par exemple, les trois premières étapes de la voie de la biosynthèse *de novo* des pyrimidines qui sont sous la dépendance de trois gènes non liés chez E.coli, sont catalysées par une seule enzyme codée par un gène résultant de la fusion de trois gènes ancestraux distincts.

L'invention concerne aussi l'association de la fluorocytosine ou le fluorouracile et de l'AZT pour une chimiothérapie combinée des tumeurs par l'expression de deux gènes suicide, sélectionnés pour leur complémentarité dans leur mode d'action et exprimés dans un vecteur eucaryote.

La société demanderesse a trouvé que la carence en dTTP par la FC activé par le gène suicide du premier groupe, permettait à l'AZT ou à un des analogues de nucléosides pyrimidiques activés par un gène suicide du second groupe d'être incorporé dans l'ADN dans de plus grandes quantités. Comme conséquence, des cellules tumorales transformées par deux gènes suicide, un du premier groupe et l'autre du second groupe, devenaient très sensibles à l'effet létal d'un traitement simultané avec de la FC ou du FU et de l'AZT ou un des autres analogues de nucleosides pyrimidiques.

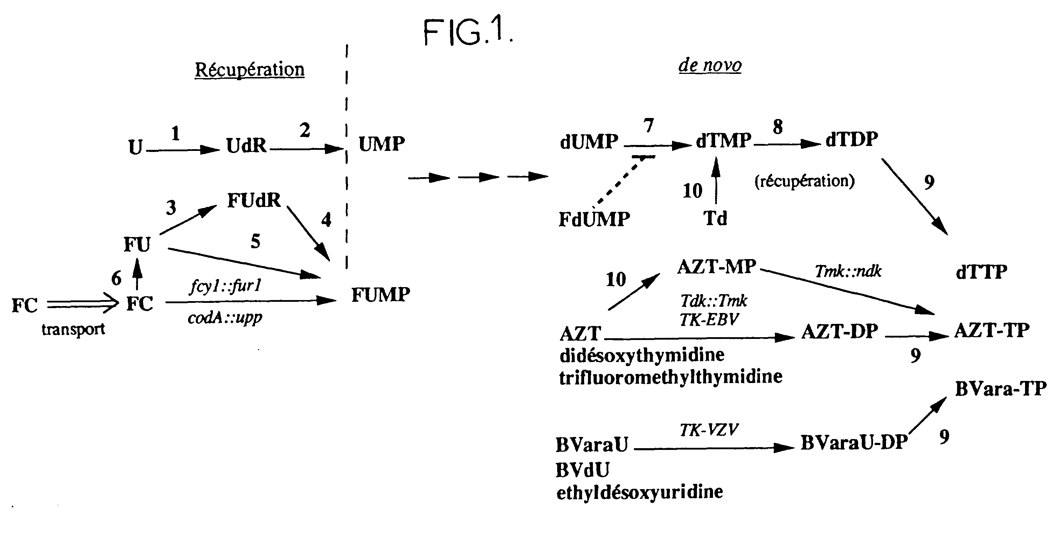

L'antimétabolite AZT-TP entre en compétition avec le nucléotide naturel dTTP, avec cependant une faible efficacité d'incorporation dans l'ADN par les polymérases cellulaires. Plus le rapport de concentration AZT-TP/dTTP est élevé, plus grande est la quantité d'AZT-TP incorporé dans l'ADN. Le bloquage de la synthèse de *novo* du dTTP n'interférant pas avec la formation d'AZT-TP, devrait modifier ce rapport, tendant à une plus grande incorporation de l'analogue de la thymidine dans les brins d'ADN, stoppant ainsi son élongation. La fluorocytosine, activée par la cytosine désaminase et la FC ou le FU activés par la cytosine désaminase associée avec une UPRTase, sont métabolisées en produit final FdUMP qui agit comme inhibiteur suicide de la thymidylate synthétase, une enzyme clé dans les voies de biosynthèse du dTTP. L'association fluorocytosine et AZT agit en synergie pour tuer les cellules traitées exprimant deux gènes suicide qui activent les deux prodrogues. Ce mécanisme est illustré dans la Figure 1.

Le nucléotide dTTP est un métabolite essentiel finement régulé dans toute cellule. Une carence sévère de dTTP par l'action d'inhibiteurs ou induit par des mutations entraîne la mort des cellules, probablement par l'induction d'une endonucléase responsable de la cassure de l'ADN double brin. L'utilisation de la FC ou du FU, activée par un gène codant pour des activités CDase et UPRTase, a pour but de tuer des cellules cibles par absence de thymidine due au manque de dTTP. La thymidine lève l'effet toxique de la FC par production de dTTP en court-circuitant le bloquage de la thymidylate synthétase via la voie de récupération des pyrimidines (voir Fig.1). L'utilisation des analogues de la thymidine ou de la desoxyuridine en association avec la FC ou le FU pour le traitement de cellules exprimant deux gènes suicide activateurs, a un effet opposé. L'incorporation d'analogues triphosphate dans l'ADN lors de la réplication et du processus de réparation est facilité par le manque de dTTP compétiteur, entraînant un effet létal plus prononcé.

L'invention comprend donc un vecteur eucaryote comprenant deux unités d'expression de gènes suicide, le premier permettant la sensibilisation des cellules tumorales à la 5-fluorocytosine ou au 5-fluorouracile et le second permettant la sensibilisation des cellules de mammifères ou infectées par le virus VIH à l'azidothymidine de manière synergique.

Selon une réalisation préférentielle de l'invention, le vecteur eucaryote comprend une première unité d'expression d'un gène suicide constituée par un gène codant pour une activité uracile phosphoribosyltransférase ou un gène chimère formé de la fusion de deux gènes, chacun pouvant être d'origine bactérienne ou fongique, codant pour une protéine unique possédant une double activité cytosine désaminase et uracile phosphoribosyltransférase.

Les gènes bactériens sont, de préférence, le gène *codA* encodant une cytosine désaminase et le gène *upp* encodant l'uracile phosphoribosyl transférase d'*Escherichia coli,* et les gènes fongiques sont le gène *fcy1* encodant la cytosine désaminase et le gène *fur1* encodant l'uracile phosphoribosyltransférase de *Saccharomyces cerevisiae.*

La seconde unité d'expression d'un gène suicide de l'invention comprend un gène hybride formé par la fusion du gène *tmk* et du gène *tdk* encodant respectivement la thymidylate kinase et la thymidine kinase d'*E.coli,* ledit gène hybride codant pour une protéine unique à double activité thymidine kinase et thymidylate kinase. Selon l'invention, un autre candidat pour la seconde unité d'expression d'un gène suicide est un gène hybride formé par la fusion d'un gène *tmk* modifié d'*Escherichia coli* et d'un gène *ndk* encodant la nucléoside diphosphokinase d'*Escherichia coli* et codant pour une protéine unique à double activité thymidylate kinase et thymidine diphosphokinase, de préférence en fusion avec le gène *ble Sh.* Selon l'invention, une seconde unité d'expression d'un gène suicide, peut également contenir le seul gène *tmk* d'E.coli ou un gène *tk* modifié provenant du virus humain Varicella-Zooster exprimant une double activité thymidine kinase et thymidylate kinase, le dit gène modifié conférant la résistance à la zéocine ou un gène similaire provenant du virus humain d'Epstein-Barr.

L'invention concerne également un médicament comprenant un vecteur eucaryote contenant les deux groupes de gènes suicide ou l'un des groupes seul.

L'invention concerne également un médicament pour le traitement des cellules tumorales sensibles à la 5-fluorocytosine et/ou au 5-fluorouracile comprenant un vecteur eucaryote contenant les deux groupes de gènes suicide ou l'un des groupes seul.

L'invention concerne également un médicament pour le traitement des cellules de mammifères ou des cellules infectées par le VIH comprenant un vecteur eucaryote contenant les deux groupes de gènes suicide ou l'un des groupes seul.

La Figure 1 décrit la voie anabolique de la 5-fluorocytosine pour donner le produit final, le FdUMP, inhibiteur spécifique de la thymidylate synthetase, une enzyme clé dans la biosynthèse *de novo* du thymidylate dTTP. Une carence en dTTP dans des cellules en division par l'action du FC activé par une enzyme à double activité CDase et UPRTase codée par les nouveaux gènes suicide du premier groupe, objet de cette invention, induit une mort appelée "mort par manque de thymidine" probablement par des mécanismes liés à l'apoptose.

Les étapes catalysées par des produits des principaux nouveaux gènes suicide du second groupe décrits dans l'invention, dans l'anabolisme de quelques analogues non toxiques de la thymidine et de l'uridine sont aussi indiquées. Ces analogues sont transformés par l'action des nouveaux gènes suicides en dérivés triphosphate qui sont incorporés dans l'ADN, inhibant ainsi l'ADN.

Les Figures 2a, 2b et 2c illustrent les plasmides d'expression contenant les gènes suicide et leur filiation.

Les Figures 3a₁, 3a₂, 3b₁ et 3b₂ présentent les cartes plasmidiques des vecteurs d'expression pZEO-SGl ; pZEO-SG2 ; pZEO-SG3 ; pZEO-SG4 contenant deux gènes suicides.

Les exemples suivants, non exhaustifs, sont présentés dans un seul but d'illustration.

### Exemple 1

### Isolement de mutants tdk codA upp d'une souche K12 d'Escherichia coli

Les bactéries *Escherichia coli* K12 métabolisent la FC, le FU et l'AZT, analogues toxiques des nucléobases et nucléosides pyrimidiques, sous l'action des enzymes CDase, UPRTase et thymidine kinase respectivement. Les souches déficientes pour l'une de ces activités sont résistantes à l'analogue correspondant. Dans le but de faciliter le clonage des allèles fonctionnels tdk⁺ et upp⁺, un mutant de la souche MC1061, déficient pour l'activité thymidine kinase, a été isolé sur la base de la résistance à l'AZT. Puis à partir de ce mutant, un mutant déficient pour l'UPRTase a été isolé par sélection de clones résistants au FU. Une culture d'*E.coli* MC1061, ayant poussé toute une nuit, dans du milieu LB, a été étalée sur des boîtes contenant du milieu M9-CA complémenté avec 5 µg ml⁻¹ d'AZT. Un mutant MC1061 *tdk* a été isolé en repiquant un clone parmi les nombreuses colonies résistantes. Une culture de ce clone, dans du milieu LB, ayant poussé une nuit à 37°C, a été bloquée à 4°C pendant 2 heures, puis 10⁸ cellules ont été étalées sur des boites M9-CA complémentées avec 30 mg ml⁻¹ de 5-fluorouracile. Vingt colonies individuelles, ayant apparu après 60 heures d'incubation, ont été repiquées sur le même milieu sélectif. Une suspension cellulaire a été préparée à partir des clones et des cellules ont été déposés sur des boites M9-CA contenant des concentrations croissantes de 5-fluorocytosine ou 5-fluorouracile. Un clone résistant à 100 µg ml⁻¹ de fluorouracile et à 1000 µg ml⁻¹ de fluorocytosine a été retenu et désigné CL108. Par la suite, une mutation dans le gène pyrD, qui confère une auxotrophie pour les pyrimidines, a été introduite dans CL108 par transduction P1, décrite par Miller [ Miller J.H. *Experiments in Molecular Genetics Cold Spring Harbor, New York, Cold Spring Harbor Laboratory Press* (1972) ]. La souche résultante CL109 est incapable d'utiliser la cytosine ou l'uracile comme seule source de pyrimidine. Les gènes *codA* et *codB,* codant respectivement pour la cytosine déaminase et la cytosine perméase, forment un opéron qui est étroitement lié à l'opéron *lac* à 8 minutes sur le chromosome *d'Escherichia coli* [ Austin E.A. & Huber B.E. *J Bacteriol 175, 3685-6* (1993); Danielsen et coll. *Mol Microbiol 6, 1335-44* (1992)]. Les souches d'*E.coli* utilisées pour les expériences de complémentation *lac Z,* basées sur la coloration au XGal, telle que MC1061, portent des délétions de différentes longueurs incluant l'opéron *codBA* en aval du gène *lac Z* et de ce fait sont dépourvues d'un système de transport actif de la cytosine. Dans le but de construire une souche dérivée de MC1061 avec un système de transport actif de la cytosine, et par conséquent de la fluorocytosine, la région comprenant le gène *codB* et son promoteur a été sous-clonée à partir de pSD112 et introduite dans pAPT110, un vecteur portant l'origine de réplication P15a [ Cornet et coll. *J Bacteriol 176, 3188-95* (1994)]. Le plasmide résultant pAPT codB, sélectionnable par la kanamycine et compatible avec les plasmides basés sur le replicon colE1, a été introduit dans la souche CL108 comprenant ou non un des plasmides pUT du Tableau 1. Le génotype des trois souches sont les suivants: MC1061 *(codA codB);* CL108 (*codA codB tdk upp*); CL109 *(codA codB tdk upp pyrD*).

Les concentrations minimales inhibitrices (CMI) des cellules aux FC, FU et AZT ont été déterminées sur des boîtes contenant du milieu minimum M9 [ Miller J.H. Experiments in Molecular Genetics Cold Spring Harbor, New York, Cold Spring Harbor Laboratory Press (1972)], complémenté avec 0,5 % d'acide Casamino, Difco, 0,2 % de glucose, 1 µg ml⁻¹ de thiamine et 1,5 % d'agar Difco (M9-CA). Quand nécessaire, des antibiotiques ont été ajoutés aux concentrations suivantes: ampicilline (Ap, 50 µg ml⁻¹), kanamycine (Km, 25 µg ml¹ ), tetracycline (Tet, 10 µg ml⁻¹) et zéocine (Zéo, 20 µg ml⁻¹). La Zéocine a été fournie par CAYLA. Les cellules d'une culture stock ont été étalées sur des boites contenant du milieu M9-CA et les antibiotiques appropriés. Après une nuit d'incubation à 37°C, des suspensions cellulaires sont préparées en resuspendant, dans du milieu M9-CA, environ 10⁷ bactéries ml⁻¹. Les cellules ont été déposées (5 ml) sur la surface de boîtes contenant du milieu M9-CA supplémenté avec des concentrations croissantes de l'analogue. La croissance des cellules a été relevée après 48 heures d'incubation. Les résultats sont montrés au Tableau 1 ci-dessous.

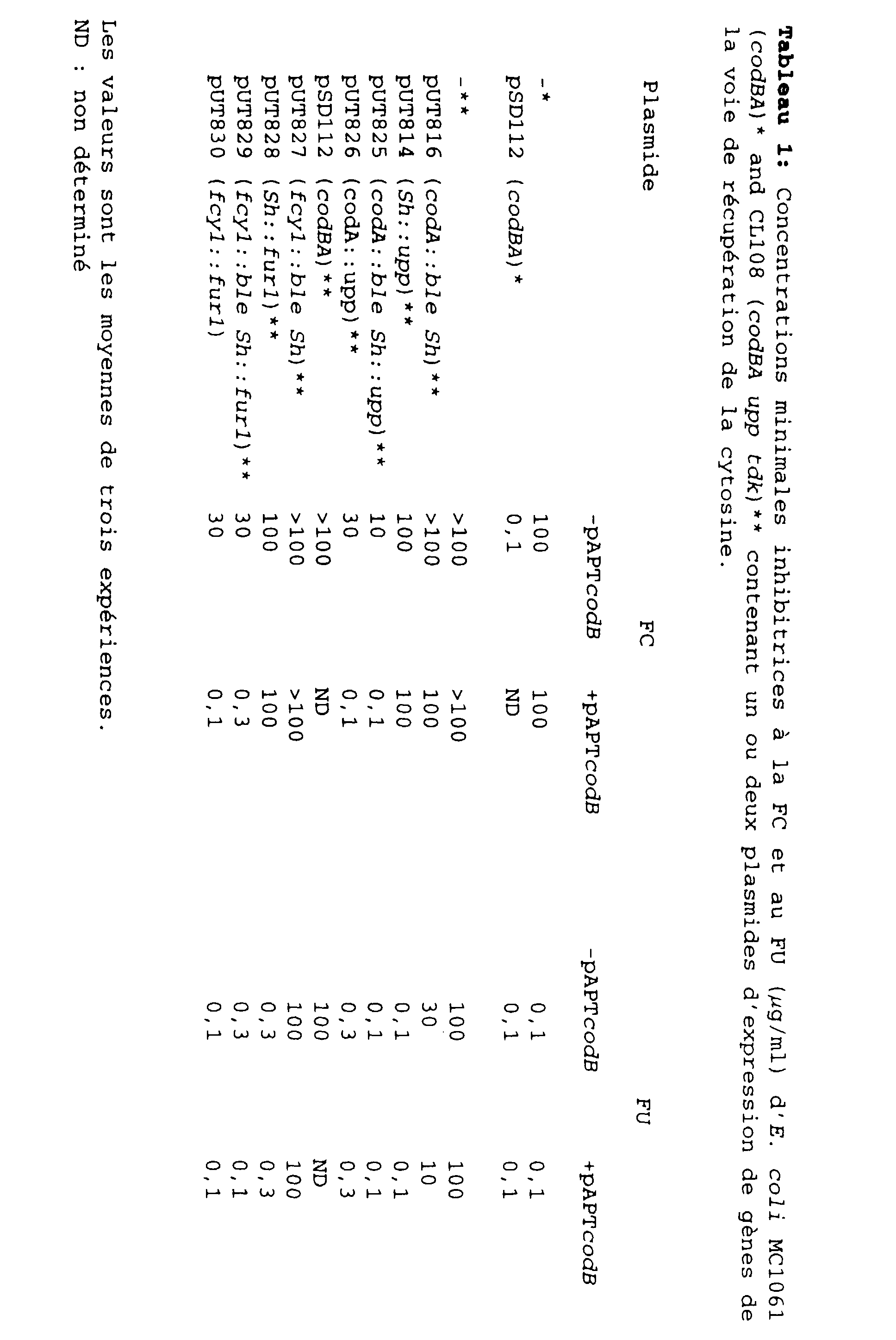

### Exemple 2

### Construction du gène codA::upp

Les séquences des gènes *codA* et *upp* d'*E.coli* ont été publiées récemment [ Danielsen et coll. *Mol Microbiol 6, 1335-44* (1992); Austin E.A. & Huber B.E. *J Bacteriol 175, 3685-6* (1993); Andersen et coll. *Eur J Biochem 204, 51-6* (1992)]. Grâce aux techniques de PCR (Polymérase chaîne réaction), le gène *codA* a été amplifié à partir du plasmide pSD112 et le gène *upp* à partir du chromosome de la souche MC1061 et introduit dans les plasmides pUT687 et pUT614 ras respectivement. Ces deux vecteurs dérivent de pUC19 par le remplacement du promoteur *lacZ* et la séquence de l'α peptide par un fragment BamHI portant le gène de sélection *ble* Sh qui code pour la résistance à la zéocine et à la phléomycine à la fois chez *E.coli* et les cellules de mammifères [ Drocourt et coll. *Nucleic Acids Res 18, 4009* (1990)] [US 5, 021, 344] [US 5, 118, 620]. Dans pUT687, le gène *ble Sh* est fusionné en phase à son extrémité 5' au gène de la thymidine kinase d'*Escherichia coli* (voir exemple 5). Dans pUT614 ras, le gène *ble Sh* est fusionné en phase du côté 3' à une séquence de 47 pb codant pour un bras de fusion polypeptidique flexible et les 12 derniers acides aminés du gène ras humain. Le promoteur bactérien synthétique EM7 et le promoteur animal pTK de pMC1néo [Thomas K.R. & Capecchi M.R. Cell 51, 503-12 (1987)] en tandem permettent l'expression du gène hybride *zéo* à la fois chez *E.coli* et les cellules de mammifères. Le gène *codA* a été amplifié par PCR en utilisant les amorces suivantes: extrémité 5'   5'   GTACCATGGTGTCGAATAACGCTTTACAAAC   3' et

extrémité 3'   5' CTCCTAGGCGTTTGTAATCGATGGCTTCTGGCTG   3'

Le fragment PCR a été coupé par NcoI et AvrII puis inséré à la place du fragment NcoI-AvrII contenant le gène tk- *E.coli* de pUT687 pour donner pUT816.

Le gène *upp* a été amplifié par PCR en utilisant les amorces suivantes: extrémité 5' 5' GTCATGCATCAAGATCGTGGAAGTCAAACACCCA 3' et

extrémité 3' 5' CTGGTCGACAAGCTTATTTCGTACCAAAGATTTTGTCACC 3'

Le fragment PCR résultant a été coupé avec NsiI et SalI et inséré dans pUT614 ras à la place du fragment NsiI-SalI contenant l'extrêmité ras pour donner pUT814. L'utilisation de CL109 a fourni une méthode de sélection positive pour le clonage des gènes *codA* et *upp* respectivement. Les transformants possédant le plasmide pUT 816 qui exprime *codA::Sh* ont été sélectionnés à partir de CL109 sur du milieu contenant la cytosine comme seule source de pyrimidine alors que les transformants possédant le plasmide pUT 814 qui exprime *Sh::upp* ont été isolés sur du milieu contenant de l'uracile. Les deux plasmides pUT 816 et pUT 814 ont été par la suite recombinés *in vitro* en ligaturant un fragment BglII-SgrAI d'un des plasmides au fragment complémentaire BglII-SgrAI de l'autre plasmide pour donner pUT825. Le gène résultant *codA::ble Sh::upp* code pour une protéine hybride qui possède trois domaines distincts séparés par des bras flexibles (Fig. 2c). A partir d'un extrait protéique de la souche Cl108(pUT825) une bande unique correspondant à une protéine de 75 kD, légèrement inférieure à la taille attendue de 80 kD pour le produit du gène *codA::ble Sh::upp* a été détectée par immuno-détection avec un antisérum de la protéine Sh . La fonctionnalité du gène de fusion *codA::ble Sh::upp* a été confirmée par la capacité de pUT825 à permettre la croissance de la souche CL109 sur un milieu contenant de la cytosine ou de l'uracile comme seule source de pyrimidine et à sensibiliser les cellules à la FC et au FU (Tableau 1). De plus pUT825 confère aux cellules réceptrices une résistance jusqu'à 200 µg ml⁻¹ de zéocine, niveau de résistance identique à ceux obtenus avec tous les plasmides des Figures 2a, 2b et 2c. Ceci correspond à une augmentation d'un facteur 100 de la résistance par rapport aux souches non transformées.

Enfin, le gène *codA::upp* (pUT825) a été construit en remplaçant le fragment AvrII- NsiI de pUT829, contenant le gène *ble Sh*, par un oligonucléotide double brin codant pour un bras de fusion peptidique flexible.

5′ CTAGGGATCTCAGGCCTTAATGGCGTATGCA 3′
CCTAGAGTCCGGAATTACCGCAT

### Exemple 3

### Construction du gène fcy1::fur1

Les séquences des gènes *fcy1* et *fur1* de *S.cerevisiae,* codant respectivement pour la cytosine désaminase et l'uracile phosphoribosyltransférase, ont été publiées [(EP 90306131.5; Kern et coll. *Gene 88, 149-57* (1990)]. Les deux gènes ont été amplifiés par les techniques de PCR à partir de l'ADN de la souche de levure OL1. Les sites de restriction NcoI-AvrII et NsiI-SalI ont été créés aux extrêmités 5' et 3' des séquences codantes des gènes *fcy1* et *fur1* respectivement en utilisant les oligonucléotides amorces suivants:
pour le gène *fcy1* extrémité 5' 5' GTACCATGGTGACAGGGGGAATGGCAAGCAA 3' et extrémité 3′ 5′ TCCCTAGGGCCTCACCAATATCTTCAAACCAATCCTG 3′ pour le gène *fur1* extrémité 5′ 5′ TGGATGCATGAACCCGTTATTCTTTTGGCTTCT 3′ et

extrémité 3'   5'   TCGAGGTCGACTTTAAACACAGTAGTATCTGTCACCAAA   3'

Le gène *fcy1* a été par la suite introduit dans pUT687 au niveau des sites NcoI et AvrII et le gène *fur1* dans pUT614 ras au niveau des sites NsiI et SalI pour générer les plasmides pUT827 et pUT828 respectivement. Les transformants d'*E.coli* possédant pUT828 (*fcy1::ble Sh*) ont été sélectionnés sur du milieu minimum contenant de la cytosine comme seule source de pyrimidine alors que les transformants possédant pUT828 (*Sh::fur1*) ont été sélectionnés sur du milieu minimum contenant de l'uracile.

Les deux gènes, *fcy1::ble Sh* et *ble Sh::fur1,* ont été ensuite recombinés en un gène unique *fcy1::ble Sh::fur1* en ligaturant un fragment NcoI-SgrAI de pUT827 au fragment complémentaire NcoI-SgrAI de pUT828, pour donner le plasmide pUT829. La fonctionnalité du gène hybride *fcy1::ble Sh::fur1* a été contrôlée par la capacité de la souche CL109, transformée par pUT829, à pousser sur du milieu contenant soit de la cytosine soit de l'uracile comme seule source de pyrimidine et par la sensibilité de la souche CL108 transformée par pUT829 à la FC et au FU (Tableau 1).

Enfin, le gène *fcy1::fur1* a été construit par une technique similaire à celle du gène *codA::upp* en remplaçant un fragment AvrII-NsiI de pUT829 par un oligonucléotide double brin codant pour un bras de fusion peptidique flexible (décrit dans l'exemple 2) pour donner pUT 830.

Les souches bactériennes transformées par pUT829 et pUT830 ont été testées pour les activités CDase et UPRTase. Les cellules récupérées à partir d'une culture de la nuit de 20 ml de milieu LB ont été lavées une fois avec un volume égal de Tris-HCl 20mM, pH 7,5 puis resuspendues dans 1 ml du même tampon contenant 1 µg ml⁻¹ de lysozyme. Après 10 minutes dans la glace, la suspension cellulaire a été l'objet de 2 cycles de congélation-décongélation puis soniquée cinq minutes par des impulsions de 30 secondes. Les débris cellulaires et les cellules intactes ont été éliminés par centrifugation et les surnageants ont été utilisés pour les dosages enzymatiques. Les dosages *in vitro* de la cytosine désaminase et de l'UPRTase ont été réalisés en suivant les protocoles décrits légèrement modifiés [Andersen et coll., (1989)]. Les mélanges réactionnels standard correspondaient à un volume de 100 µl: Tris-HCl 50 mM pH 7,5; DTT 1 mM; 10 µl d'extraits cellulaires pour les deux activités plus de la cytosine (5 mM) pour le dosage de la cytosine désaminase plus de l'uracile 5 mM, MgCl2 5mM et PRPP 2 mM (Sigma) pour celui de l'UPRTase. Les réactions enzymatiques, après 2 heures d'incubation à 30°C, ont été stoppées par refroidissement dans de la glace. Des aliquots de 10 µl ont été déposés sur des plaques en couche mince de cellulose imprégnée de polyethylenimine (Schleicher & Schuell) ainsi que 4 µg de cytosine, uracile et UMP comme témoins. Les plaques en couche mince ont été révélées dans l'eau pour séparer la cytosine et l'uracile de l'UMP, ou dans un mélange butanol-1/H2O (86/14) pour obtenir une séparation nette entre la cytosine et l'uracile. La présence de taches, correspondant au produit formé, visibles sous les UV, ont permis de décéler l'activité enzymatique. Les transformations de la cytosine en uracile et l'uracile en UMP n'ont été observées que dans les extraits cellulaires *d'E.coli* transformés par pUT829 ou pUT830.

### Exemple 4

### Clonage du gène tmk d'Escherichia coli

Le locus génétique de tmk, le gène codant pour la dTMP kinase, a été positionné autour de 24 minutes sur la carte chromosomique d'*E.coli* [ *Binkley* J.P. et Kuempel P.L. *J Bact 168, 1457-8* (1986)]. A partir de l'analyse des séquences nucléotidiques, déposées à Genbank, positionnées entre la 24^{ème} et 25^{ème} minute de la carte génétique, il est possible qu'une seule région non séquencée comprise entre les gènes *acpP* et *holB* pouvait contenir le gène *tmk*. En conséquence, la région intergénique acpP-holB a été amplifiée par PCR à partir du phage 1 E9G1[236] de la banque de Kohara [Kohara et coll. Cell 50, 495-508 (1987)] et cloné. Les amorces oligonucléotidiques utilisées pour la PCR ont été:

En 5′ terminal 5′ ATTTCGAATTCCCTCCCTGGAGGACAAACGTGT 3′

En 3′ terminal 5′ CCACCGGTACCATCTCATGCGTCCAACTCCTTC 3′

L'amorce 5' terminale contenait 25 bases homologues (en gras) à l'extrémité 3' de la séquence publiée du gène *acpP* [Rawlings M. et Cronan J.J. J Biol Chem 267, 5751-4 (1992)] et des bases additionnelles pour créer un site EcoRI. L'amorce 3' terminale contenait 26 bases homologues (en gras) à la séquence holB [Carter et coll. J Bacteriol 175, 3812-22 (1993); Dong et coll. J Biol Chem 268, 11758-65 (1993)] autour du codon d'initiation ATG et des bases additionnelles pour créer un site Asp718I.

Le fragment d'ADN obtenu après amplification PCR (~ 4 kb) a été digéré par EcoRI at Asp718I et inséré dans pZEOSVl (n° accession Genbank L36849; commercialisé par CAYLA) ouvert par EcoRI et Asp718I. Après transformation d'*E.coli* par le mélange de "ligation", il n'a pas été possible d'obtenir des transformants contenant la taille attendue du plasmide recombinant suggérant que la région amplifiée était soit toxique soit létale pour E.coli. Le même résultat a été obtenu après clonage de la région amplifiée réduite à un fragment BglII-Asp718I de 2,5 kb dans pUT58 (commercialisé par CAYLA, digéré par BglII et Asp718I). Les sous-clonage ultérieurs d'un fragment BglII-PstI de 1,6 kb dans pUT106 (commercialisé par CAYLA, digéré par BglII et PstI) ou d'un fragment PstI-Asp718I de 0,9 kb dans pAPT 110 (digéré par PstI et Asp718I), ont permis d'obtenir des plasmides recombinants stables appelés respectivement pUT126 et pUT125.

Des amorces oligonucléotidiques ont été synthétisées sur la base des vecteurs de clonage pUT106 et pAPT110 pour initier le séquençage des fragments insérés dans le sens PstI vers Asp718I pour pUT125 et PstI vers BglII pour pUT126. Les séquences obtenues à l'aide de ces amorces ont été utilisées pour définir de nouvelles amorces et ainsi poursuivre le sequençage des fragments insérés. Cette stratégie a permis d'obtenir des informations concernant la séquence des deux brins de 1288 paires de base. La séquence contenait une région codante de 642 nucléotides qui encode une protéine de près de 25 kD. La comparaison des séquences protéiques des dTMP kinases humaine, de levures ou des virus de la vaccine, de la variole ou de la fièvre du porc africain avec cette protéine d'*E.coli* déduite de la séquence nucléotidique, démontrait une forte homologie, indiquant bien que le gène de la dTMP kinase d'*E.coli* a été cloné.

Le codon stop du gène *tmk* d'*E.coli* et le codon d'initiation proposé pour holB [Carter et coll. J Bacteriol 175, 3812-22 (1993); Dong et coll. J Biol Chem 268, 11758-65 (1993)] sont imbriqués suggérant un couplage traductionnel de ces deux gènes, formant un opéron. La séquence d'une phase ouverte de lecture, située en amont du gène *tmk,* a été analysée par comparaison avec les squences déposées dans la banque de données GenBank. Une identité parfaite (100% d'homologie tant au niveau des acides aminés que des acides nucléiques) a été trouvée avec une protéine hypothétique d'*E.coli* mentionnée dans la région 3' du gène *pabC*, positionné au préalable à 25 minutes sur le chromosome d'*E.coli* [Gren et coll. J Bacteriol 174, 5317-23 (192)]. En fonction de ces résultats, il a été conclu que le gène *tmk* était situé juste avant le gène *holB* (24,95 min) et juste après la séquence génétique (dans le sens des aiguilles d'une montre sur la carte génétique) acpP-fabF-pabC-orfX.

Pour faciliter les expériences de sous-clonage, le gène *tmk* d'*E.coli* a été modifié par PCR aux extrémités 5' et 3' terminales. Les sites NcoI et MscI ont été introduits au début de la séquence codante du gène *tmk* pour faciliter la construction de fusions de gènes en 5' et optimiser la séquence d'initiation eucaryotique. La région C-terminale a été modifiée par l'introduction d'un site MluI, juste avant le codon stop, pour permettre la construction de fusions de gènes en 3', et d'un site RsrII juste derrière le codon stop modifié pour faciliter le sous-clonage. La séquence *tmk* du pUT125 a été utilisée comme matrice pour la réaction d'amplification PCR à l'aide des amorces nucléotidiques suivantes:

En 5′ terminal 5′ AGGACCATGGCCAGAAGTAAGTATATCGTCATTGAGGGG 3′

En 3' terminal 5' TGCACGGACCGTTACGCGTCCAACTCCTTCACCCAGTGG 3'

L'activité enzymatique codée par le gène tmk d'*E.coli* a été démontrée par complémentation fonctionnelle d'un mutant dTMP kinase thermosensible de levure (*cdc8*) à température non "permissive". Le fragment d'ADN amplifié par PCR contenant le gène *tmk* modifié a été digéré par MscI et RsrII et introduit dans le vecteur navette *E.coli*-levure pUT377 (commercialisé par CAYLA) digéré par MscI et RsrII. Le mélange de ligation a été utilisé pour transformer *E.coli* afin de préparer l'ADN du plasmide recombinant (pUT391). La souche *Saccharomyces cerevisiae* CMY 616 (*MAT* α *ura-3 leu-2 his-7 cdc-8 can 1-100*) a été transformée avec pUT391. Tous les transformants ura⁺' selectionnés à 22°C, ont été ensuite repiqués sur des boites de milieu YEPD et testés pour leur croissance à 32°C (température non permissive du mutant cdc8). Tous ont poussés à 32°C démontrant le phénotype tmk⁺. Quelques uns d'entre eux ont été étalés sur boites de milieu non sélectif pour obtenir des colonies ura⁻ qui sont devenues sensibles à la température (phénotype tmk), indiquant que la propriété de pousser à 32°C était bien corrélée à la présence du plasmide pUT391.

### Exemple 5

### Construction des gènes tdk::tmk et tmk::ndk

La séquence du gène *tdk* d'*E.coli* a été récemment publiée [Bockamp et coll. *Gene 101, 9-14* (1991); Black M.E. et Hruby D.E. *Mol Microbiol 5, 373-9* (1991)]. La région codante de la thymidine kinase a été amplifiée par PCR en utilisant l'ADN chromosomique de la souche d'*E.coli* DH5α comme matrice et les amorces nucléotidiques suivantes (bases homologues en gras):

En 5' terminal 5' GTACCATGGCACAGCTATATTTCTACTAT 3'

en 3' terminal 5' CTGGATCCCTAGGTCGTGGCGATGCCTTTCCTGAATAGCC 3'

Le fragment PCR obtenu a été coupé par NcoI et BamHI puis inséré à la place du fragment NcoI-BclI de pUT599 (préparé à partir d'une souche d'*E.coli* dam⁻) contenant le gène *tk-HSV1* pour donner pUT687.

Le fragment d'ADN obtenu par PCR contenant le gène *tmk* d'*E.coli* modifié (exemple 4), digéré par NcoI et MluI (traité par Klenow), a été inséré dans pUT655 (dérivé de pUT599, commercialisé par CAYLA) ouvert au niveau des sites NcoI et PvuII pour donner pUT832.

Le fragment PCR contenant le gène *tmk* d'*E.coli* modifié (exemple 4), digéré par NcoI et RsrII a été inséré à la place du fragment NcoI-RsrII de pUT599 contenant la fusion des gènes *tk-HSV1* et *ble Sh* pour donner pUT836.

Le fragment EcoRV-NotI de pUT832 contenant la fusion *tmk(E,coli)::ble Sh* a été inséré dans pUT833 coupé par EcoRV et NotI pour donner pUT834.

La séquence du gène *ndk* d'*E.coli* a été récemment publiée [Hama et coll. *Gene 105, 31-6* (1991)]. La région codante du gène *ndk* a été amplifiée par PCR à partir de l'ADN chromosomique de la souche d'*E.coli* DH5α à l'aide des amorces nucléotidiques suivantes (bases homologues en gras):

En 5′ terminal 5′CCATATGCATGGCTATTGAACGTACTTTTCCATCATCAAA 3′

En 3′ terminal 5′ TGTGTCGACTTAACGGGTGCGCGGCACACTTCGCC 3′

Le fragment PCR obtenu a été coupé par NsiI et SalI puis inséré dans pUT614 ras à la place du fragment NsiI-SalI contenant le gène *ble Sh::ras* pour donner pUT692.

Le fragment Nco-SgrAI de pUT832 contenant la fusion *tmk(E.coli)::ble Sh* a été inséré dans pUT692, coupé par NcoI et SgrAI pour donner pUT835.

Dans pUT837, le gène *tmk* d'*E.coli* est fusionné en 3' en phase avec une séquence de 18 paires de base, codant pour un bras polypaptidique flexible, et le gène *ndk.* Pour faciliter cette construction, une nouvelle amorce PCR 3' terminale a été utilisée pour modifier la région codante du gène *tmk* à partir de pUT125 (bases homologues en gras):

En 5′ terminal 5′   AGGACCATGGCCAGAAGTAAGTATATCGTCATTGAGGGG 3′

En 3′ terminal 5′ GACATGCATCCAGGTCCCTGGTCCAACTCCTTCACCCAGTGGGTC
3′

Le fragment d'ADN obtenu par PCR contenant le gène *tmk* modifié du côté 3' a été digéré par NcoI et NsiI et introduit dans le vecteur pUT692 coupé par NcoI et NsiI pour donner pUT837.

La fonctionnalité des gènes hybrides contenant le gène *tdk a* été démontrée dans le mutant d'*E.coli* tdk⁻ CL108 qui devient hypersensible à l'AZT après transformation par un plasmide exprimant le gène *tdk.* La fonctionnalité des gènes hybrides contenant le gène *tmk* a été démontrée avec le mutant tmk⁻ TD205 d'*E.coli* par le niveau de résistance à la bromodésoxyuridine (30µg ml⁻¹), un phénotype associé à l'allèle *tmk* dans la souche particulière TD205 [Binkley J.P. et Kuempel P.L. *J Bacteriol 168, 1457-8* (1986)]

### Exemple 6.

### Clonage des gènes tk-VZV et tk-EBV.

Les gènes *tk* du virus de l'herpès simplex codent à la fois les activités thymidine et thymidylate kinases. Des études cinétiques ainsi que l'alignement comparatif des séquences nucléiques révèlent ques les gènes des virus Varicella-Zooster (*tk-VZV*) et d'Epstein-Barr(*tk-EBV*) pourraient eux-aussi être liés à une forte activité thymidylate kinase (Baer et al, *Nature, 310, 207-211* (1984); Lttler et al., *EMBO J. 5, 1959-1966* (1986)).

Afin de déterminer si les activité TKs de ces deux virus sont capables de phosphoryler l'AZT monophosphate, les gènes *tk* ont été clonés et exprimés dans la souche d'*E. coli* CL108. Les gènes *tk-VZV* et *tk-EBV* ont été amplifiés en utilisant des techniques de PCR à partir d'un fragment EcoRI-BamHI de 2,2 paires de kilobase inséré dans l'ADN du bactériophage mp18 M13 (fourni par Dr R. Gaillard, Burroughs-Wellcome Co, USA) dans le cas de *tk-VZV,* et du plasmide pUC8X contenant le cadre ouvert de lecture BXLF1 du virus d'Epstein-Barr (fourni par Dr Y. Connolly, Institute for Cancer Research, RU). Les séquences nucléiques étant connues (Mori et al. *Intervirology 29, 301-310* (1988); Robertson G. & Whalley J. M. *Nucl. Acids Res. 16, 11303-11317),* ils ont été amplifiés tous deux grâce aux amorces oligonucléotidiques suivantes: Extrémité 5'  5' GTACCATGGCATCAACGGATAAAACCGATGTAAAAATG 3' et Extrémité 3'  5' CGCCCTAGGGAAGTGTTGTCCTGAAC 3' pour *tk-VZV* Extrémité 5'  5' GGGAGCGCTGGATTTCCAGGAAAC 3' et Extrémité 3'  5' AAGCCTAGGTCCCGATTTCCCCTCTC 3' pour *tk-EBV*

Le fragment amplifié contenant *tk-E.coli* a été digéré par les enzymes NcoI et AvrII puis inséré à la place du fragment NcoI-AvrII contenant le gène *tk-HSV* du plasmide pUT687 afin de créer le plasmide pUT820.

Le gène *tk-EBV* a été cloné de façon similaire dans le vecteur pUT687 pour produire le vecteur pUT819. Le gène *tdk-EBV* contenant toutefois un site NcoI interne, le produit d'amplification PCR a été digéré par les enzymes Eco47III et AvrII. Le fragment NcoI-AvrII du plasmide pUT687 contenant le gène *tk-E.coli* a été remplacé par le fragment amplifié en prédigérant le vecteur pUT687 pat l'ADN polymérase de Kleenow après coupure par NcoI.

Les clones CL108 transformés par les fusions *tk-VZV::ble Sh* et *tk-EBV::ble Sh* ont été sélectionnés sur milieu riche contenant 200 µg/ml de zéocine. L'expression des protéines hybrides a été vérifiée par immunodétection avec un antisérum de la protéine Sh qui a détecté des bandes de 50 kd et 80 kd respectivement. La fonctionnalité des gènes *tk* a été montrée par l'incapacité des transformants de pousser sur un milieu LB complémenté par 10 µg/ml d'AZT.

### Exemple 7

### Construction de vecteurs d'expression porteurs de deux gènes suicides hybrides

### Etape 1 : Construction de vecteurs d'expression porteurs d'un gène suicide

La construction de vecteurs d'expression portant deux gènes suicide hybrides comprend deux étapes. Dans la première étape, une série de plasmides contenant un seul gène suicide fusionné en phase avec le gène *ble Sh* ont été construits. Tous ces plasmides dérivent du pUT 809, un plasmide obtenu par ligation du fragment AseI-BamHI de 2238 bp du pZEO-SVl (distribué par Cayla et Invitrogen, Genbank n°360849) portant le réplicon *d'E.coli* col E1, l'origine de réplication du phage F1, l'amplificateur et le promoteur du gène précoce du cytomégalovirus humain, avec le fragment AseI-BamHI de 1764 bp du pUT 599 (catalogue Cayla) contenant le gène *tk-HSV1::ble Sh.* Des cellules compétentes de la souche CL108 tdk⁻ (isolée dans l'exemple 1) ont été transformées avec le mélange de la ligation et une dilution appropriée a été étalée sur du milieu LB contenant 20 µg/ml de zeocine. L'analyse de la séquence d'ADN des plasmides des clones résistants à la zéocine incapables de croître sur le milieu LB + AZT (10 µg/ml) a confirmé la présence de pUT 809.

Ensuite le gène *tk-HSV1::ble Sh* du pUT 599 a été successivement remplacé par les quatre gènes suivants : *tk-EBV::ble Sh tk-VZV:: ble Sh* (tous deux décrits dans l'exemple 6), *tdk::tmk::ble Sh , tmk::ble Sh ::ndk* (tous deux décrits dans l'exemple 5).

Les gènes de fusion, *tk-EBV::ble Sh , tk-VZV::ble Sh* et *tdk::tmk::ble Sh* ont été isolés sous forme de fragments BbrPI-RsrII à partir des plasmides pUT838, pUT 839 et pUT 840 respectivement. Le gène *tdk::tmk::ble Sh* a été isolé sous forme d'un fragment AseI-SalI à partir de pUT 835 (Figure 2b) qui a substitué le fragment *tk-HSVl::ble Sh* contenant le fragment AseI-SalI de pUT 809 pour donner pUT 841. Comme le pUT 809, les trois plasmides recombinants pUT 837, pUT 838 et pUT 839 sensibilisent fortement la souche CL108 à l'AZT et confèrent la résistance à la zéocine.

### Etape 2 : Construction de vecteurs d'expression porteurs de deux gènes suicide hybrides

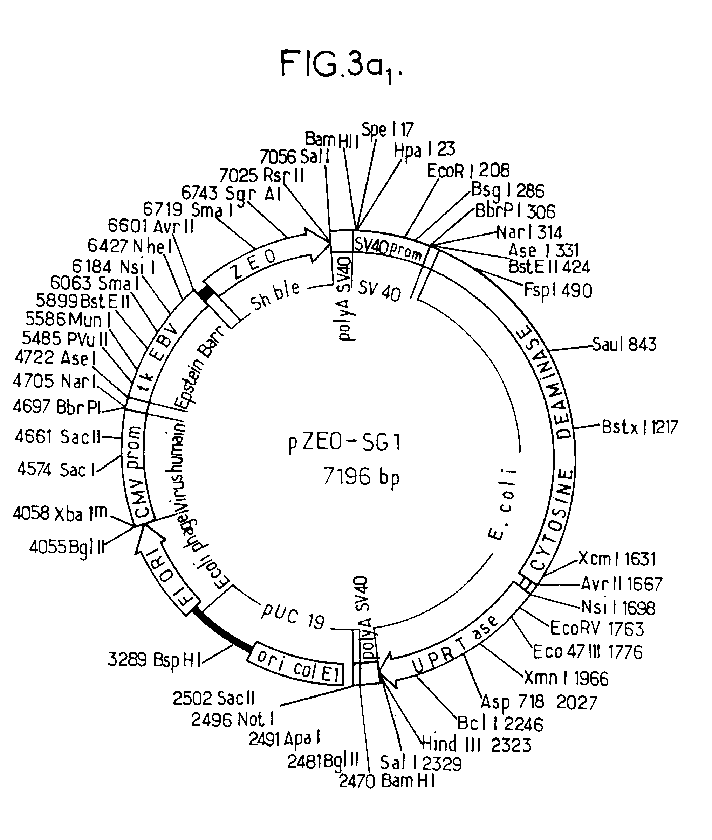

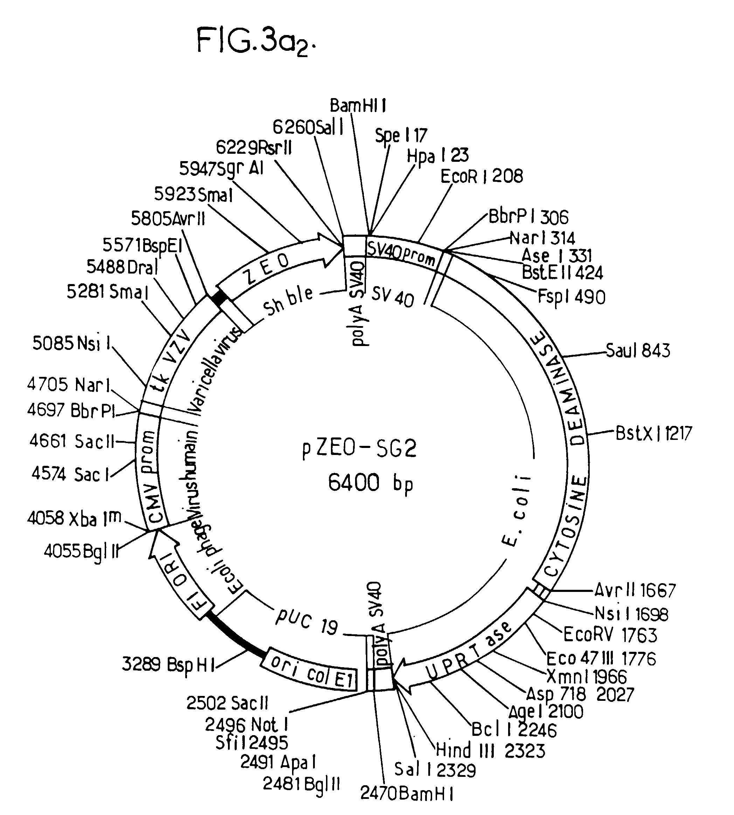

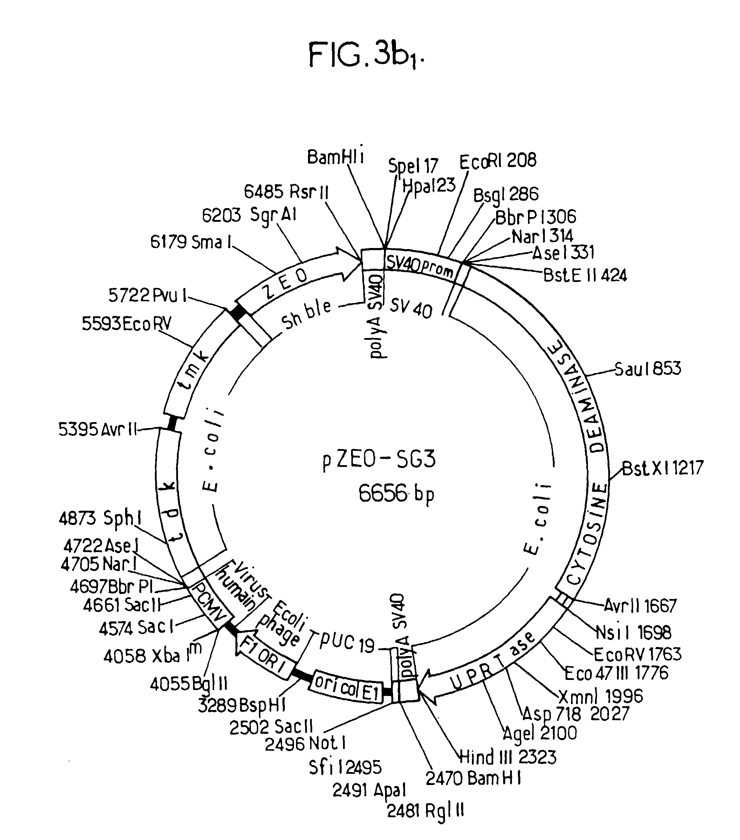

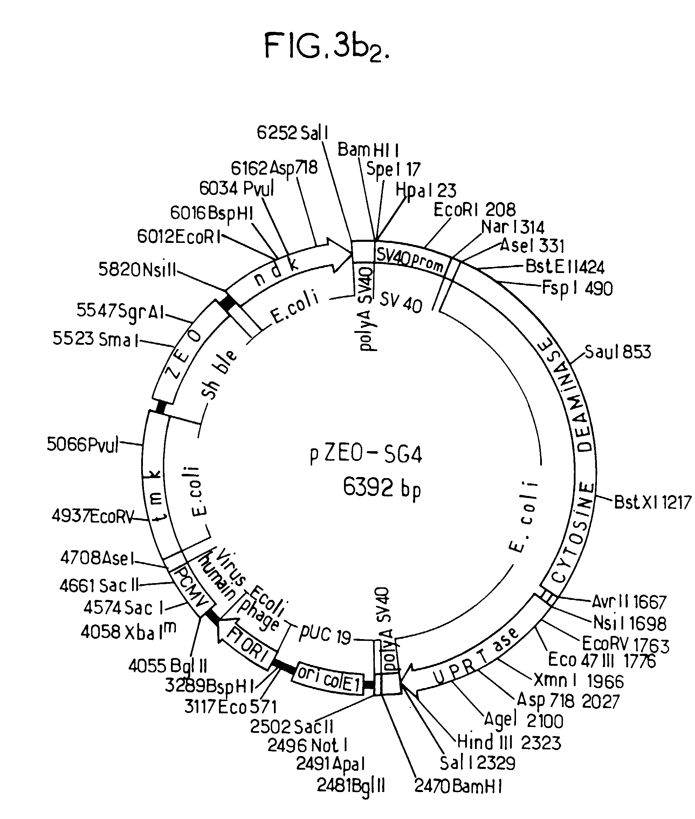

Le fragment BamHI codant pour les protéines hybrides CDase et UPRTase, isolé à partir du pUT 826 (Figure 2c) a été inséré dans le site unique BamHI des pUT 838, pUT 839, pUT 840 et pUT 841 pour donner respectivement pZEO-SG1, pZEO-SG2, pZEO-SG3 et pZEO-SG4 (Figures 3a₁, 3a₂, 3b₁ et 3b₂). La bactérie réceptrice CL108 contenant l'un des trois plasmides recombinants pZEO-SG1, pZEO-SG2, pZEO-SG3 est sensible au FC, FU et AZT et est capable de pousser sur du milieu LB contenant 200 µg/ml de zéocine.

Les séquences nucléotidiques complètes de pZEO-SG1, pZEO-SG2, pZEO-SG3 et pZEO-SG4 ont été déposées à Genbank (USA) sous les numéros d'accession L37432; L37440, L37441 et L37442 respectivement.

### Exemple 8

*Les lignées cellulaires de mélanomes B16 exprimant les gènes codA::ble Sh ::upp (ou fcy1::ble Sh::fur1 sont sensibles au FC*).

La lignée hautement métastatique de mélanome B16 de souris BL6, fournie par Dr S. Cros (Laboratoire de Pharmacologie et de Toxicologie Fondamentales, CNRS, France) a été utilisée pour déterminer l'efficacité des constructions selon l'invention dans les lignées de cellules tumorales. Les clones parentaux et recombinants ont poussé dans un milieu MB16, milieu RPMI1640 (GIBCO BRL) contenant de la L-glutamine, 10% de sérum de cheval (GIBCO BRL), 2 h/1 de bicarbonate de sodium et ajusté à pH 7.

Le protocole de transfection cellulaire fut le suivant: les cellules à l'état de subconfluence ont été incubées à 37°C dans des boîtes de Pétri d'un diamètre de 35 mm contenant un ml de milieu MB16 sans sérum, 5 pmoles d'ADN plasmidique et 10 µg de polybrène (SIGMA). Après 20 heures d'incubation, les cellules ont été traîtées pendant 3 minutes par du DMSO (Sigma) dilué 30% vol/vol dans le milieu MB16. Les cellules ont été ensuite lavées, incubées 72 heures dans le milieu MB16 puis diluées dans des boites de Pétri de diamètre 10 cm dans le milieu MB16 contenant 50 µg/ml de zéocine (CAYLA). Après 20-25 jours, des clones résistants à la zéocine sont visibles. Trois clones ont été prélevés pour chaque construction, transferrés dans des boîtes à 24 puits puis incubés 7 jours dans un milieu contenant la zéocine. La résistance à la zéocine a permis la sélection des cellules transfectées.

La conversion séquentielle de cytosine marquée radioactivement en uracile puis de l'uracile en uracile monophosphate fut suivi *in vitro* à partir de lysats cellulaires afin de mettre en évidence les activités cytosine désaminase et uracile phosphoribosyltransférase. Cet essai a été effectué selon les méthodes déjà décrites (Mullen, 1992; Natalini et al., *J. Biol. Chem. 254, 1558-1563* (1979)): environ 10⁶ cellules ont été lavées puis resuspendues dans 10 µl de Tris.HCl 100 mM pH7.8 / EDTA 1 mM / dithiothreitol 1mM. Après cinq cycles de congélation-décongélation, 10 µl de lysat cellulaire furent incubés 4 heures en présence de cytosine tritiée et d'uracile marqué au carbone 14. Ces échantillons, auxquels ont été ajoutés 0,4 mg/ml de cytosine, uracile et uracile monophosphate non marqués ont été déposés sur plaques de chromatographie couche mince (plaques Kodak 13254) puis révélés par du 1-butanol (85%). La radioactivité liée aux bandes découpées sous UV à faible longueur d'onde relative aux molécules de cytosine, uracile et uracile monophosphate a été estimée dans un compteur à scintillation.

Le Tableau 2 montre l'influence des deux activités au niveau de la toxicité à la 5FC. De plus l'expression de l'une ou l'autre de ces fonctions n'a pas d'effet négatif sur la croissance cellulaire en absence de 5FC.

**Tableau 2:** Concentrations Minimales Inhibitrices d'AZT et de 5FC (µg/ml) vis-à-vis de mélanomes B16 transfectés par des plasmides portant des gènes suicide gouvernant des voies métaboliques de la biosynthèse des pyrimidines.

| Plasmide | AZT | FC |
|---|---|---|
| | 300 | >500 |
| pUT687 *(tdk::ble Sh)* | 300 | ND |
| pUT832 *(tmk::ble Sh)* | 10 | ND |
| pUT834 *(tdk::tmk::ble Sh)* | 1 | ND |
| pUT816 *(codA::ble Sh)* | ND | 250 |
| pUT814 *(ble Sh::upp)* | ND | 500 |
| pUT825 *(codA::blesh::upp)* | ND | 5 |
| pUT827 *(fcy1::ble Sh)* | ND | 500 |
| pUT828 *(ble Sh::fur1)* | ND | 500 |
| pUT829 *(fcy1::ble Sh::fur1)* | ND | 10 |

Les valeurs indiquées sont les moyennes obtenues après trois expériences identiques.
ND: non déterminée

### Exemple 9:

### Les lignées cellulaires de mélanomes B16 exprimant les gènes tdK::tmk::ble Sh et tk-EBV::ble Sh sont sensibles à l'AZT.

Les cellules de mélanomes ont été transfectées comme décrit à l'exemple 8 en sélectionnant sur milieu MB16 contenant de la zéocine. Le Tableau 2 montre que la présence de l'activité thymidylate kinase augmente la métabolisation de l'AZT via la voie de récupération des pyrimidines d'un facteur de 300 (*tdk::tmk::ble Sh*) et de 15 (*tk-EBV::ble Sh*) respectivement.

### Exemple 10:

*Les lignées cellulaires de mélanome B16 exprimant à la fois les gènes tdk::ble Sh::tmk ou tk-EBV::ble Sh et le gène codA::upp sont très sensibles à l'AZT et au FC.*

Les cellules B16 ont été transfectés comme décrit exemple 8 en sélectionnant sur milieu MB16 contenant de la zéocine. Les plasmides utilisés ont été construits dans l'optique de bloquer la voie de biosynthèse *de novo* des pyrimidines grâce à la fusion *codA::upp* (Cf exemple 2) en présence de 5-FC et ce afin d'abaisser le taux intracellulaire de dTTP et donc d'amplifier l'effet toxique de l'AZT. Le Tableau 3 indique qu'une telle conception permet de réduire considérablement les doses létales en AZT.

**Tableau 3:** Concentrations Minimales Inhibitrices en présence d'AZT (en µg/ml) et en présence de FC (2.5 µg/ml) vis-à-vis de mélanomes B16 transfectés par des plasmides portant des gènes suicide gouvernant des voies métaboliques de la biosynthèse des pyrimidines.

| Plasmide | AZT | |
|---|---|---|
| | -FC | +FC |
| pZEO SG1 | 20 | 1 |
| pZEO SG2 | 300 | 300 |
| pZEO SG3 | 1 | <1 |
| pZEO SG4 | 10 | 1 |

Les valeurs indiquées sont les moyennes obtenues après trois expériences identiques.
ND: non déterminée

### Exemple 11:

*Les lignées cellulaires de mélanomes B16 exprimant à la fois le gène tk-VZV::ble Sh et le gène codA::upp sont très sensibles à l'AZT et au FC.*

En utilisant le même protocole que celui décrit plus haut, les résultats présentés Tableau 3 ont été obtenus.

La Figure 1 illustre le mécanisme de l'association fluorocytosine et AZT. Dans cette Figure, les abréviations suivantes sont utilisées :
- U : uracile,
- UdR : uridine,
- FU : 5-fluorouracile,
- FC : 5-fluorocytosine,
- Td : thymidine.

Les chiffres 1 à 10 correspondent aux enzymes suivantes :
1 - uridine phosphorylase
2 - uridine kinase
3 - uridine phosphorylase
4 - uridine kinase
5 - uracile phosphoribosyltransférase
6 - cytosine desaminase
7 - thymidylate synthétase
8 - thymidylate kinase
9 - nucleoside diphosphokinase
10- thymidine kinase.

## Revendications

1. Vecteur eucaryote portant deux unités d'expression d'un gène suicide, la première rendant les cellules tumorales sensibles à la 5-fluorocytosine et/ou au 5-fluorouracile et la deuxième sensibilisant les cellules de mammifères ou les cellules infectées par le VIH à l'azidothymidine de façon synergique.

2. Vecteur eurcaryote selon la revendication 1, caractérisé en ce que la première unité d'expression du gène suicide comprend un gène exprimant l'activité uracile phosphoribosyltransférase ou un gène hybride résultant de la fusion de deux gènes pouvant être d'origine bactérienne ou fongique, ledit gène hybride codant pour une protéine unique possédant à la fois les activités cytosine désaminase et uracile phosphoribosyltransférase.

3. Vecteur eucaryote selon la revendication 2, caractérisé en ce que les deux gènes bactériens sont le gène *codA* codant pour la cytosine désaminase et le gène *upp* codant pour l'uracile phosphoribosyltransférase d'*E.Coli.*

4. Vecteur eucaryote selon la revendication 2, caractérisé en ce que les deux gènes fongiques sont le gène *tcyl* codant pour la cytosine désaminase et le gène *furl* codant pour l'uracile phosphoribosyltransférase de *Saccharomyces cerevisiae.*

5. Vecteur eucaryote selon l'une des revendications 1 à 4, caractérisé en ce que la seconde unité d'expression du gène suicide comprend un gène *tmk* d'*E.Coli* codant pour la thymidylate kinase fusionné avec un gène conférant une résistance à la zéocine.

6. Vecteur eucaryote selon l'une des revendications 1 à 4, caractérisé en ce que la seconde unité d'expression du gène suicide comprend un gène hybride formé par la fusion du gène *tmk* et d'un gène *tdk* d'*E.coli* codant pour une protéine unique et portant à la fois les activités thymidine kinase et thymidylate kinase.

7. Vecteur eucaryote selon la revendication 6, caractérisé en ce que le gène hybride comprend également une fusion avec le gène *ble Sh.*

8. Vecteur eucaryote selon l'une des revendications 1 à 4, caractérisé en ce que la deuxième unité d'expression du gène suicide comprend un gène hybride résultant de la fusion d'un gène tmk d'*E.coli* fusionné avec un gène conférant une résistance à la zéocine et un gène *ndk* encodant la nucléoside diphosphokinase d'*E. coli* et codant pour une protéine unique portant à la fois les deux activités thymidylate kinase et thymidine diphosphokinase.

9. Vecteur eucaryote selon la revendication 8, caractérisé en ce que le gène hybride comprend également une fusion avec le gene *ble Sh.*

10. Vecteur eucaryote selon l'une des revendications 1 à 4, caractérisé en ce que la deuxième unité d'expression du gène suicide comprend un gène *tk* modifié du virus humain Varicella-Zoster, encodant la thymidine kinase et la thymidylate kinase, ledit gène modifié portant la résistance à la zéocine.

11. Vecteur eucaryote selon l'une des revendications 1 à 4, caractérisé en ce que la deuxième unité d'expression du gène suicide comprend un gène *tk* modifié du virus humain d'Epstein-Barr encodant la thymidine kinase et la thymidylate kinase, ledit gène modifié portant la résistance à la zéocine.

12. Vecteur eucaryote portant une unité d'expression d'un gène suicide permettant la sensibilisation des cellules tumorales à la 5-fluorocytosine et/ou au 5 fluorouracile, caractérisé en ce que l'unité d'expression du gène suicide comprend un gène exprimant l'activité uracile phosphoribosyltransférase ou un gène hybride résultant de la fusion de deux gènes pouvant être d'origine bactérienne ou fongique, ledit gène hybride codant pour une protéine unique possédant à la fois les activités cytosine désaminase et uracile phosphoribosyltransférase.

13. Vecteur eucaryote selon la revendication 12, caractérisé en ce que les gènes bactériens sont le gène *codA* codant pour la cytosine désaminase et le gène *upp* codant pour l'uracile phosphoribosyl transférase d'*E.coli.*

14. Vecteur eucaryote selon la revendication 12, caractérisé en ce que les gènes fongiques sont le gène *fcyl* codant pour la cytosine désaminase et le gène *furl* codant pour l'uracile phosphoribosyl transférase de *Saccharomyces cerevisiae.*

15. Vecteur eucaryote portant une unité d'expression d'un gène suicide permettant la sensibilisation des cellules de mammifères ou des cellules infectées par le virus VIH à l'azidothymidine ou à d'autres analogues de nucléosides pyrimidiques comme la didésoxythymidine, la trifluoromethylthymidine, l'éthyldésoxyuridine, le bromovinyldésoxyuridine, le bromovinylarabinouracile.

16. Vecteur eucaryote selon la revendication 15, caractérisé en ce que l'unité d'expression du gène suicide comprend un gène *tmk* d'*E.coli* codant pour la thymidylate kinase fusionné avec un gène conférant une résistance à la zéocine.

17. Vecteur eucaryote selon la revendication 15, caractérisé en ce que l'unité d'expression du gène suicide comprend un gène hybride résultant de la fusion d'un gène *tmk* modifié d'*E.coli* et le gène *tdk* d'*E.coli* codant pour la thymidine kinase lequel gène hybride code pour une protéine unique portant à la fois les activités thymidine kinase et thymidylate kinase.

18. Vecteur eucaryote selon la revendication 17, caractérisé en ce que le gène hybride comprend également une fusion avec le gène *ble Sh.*

19. Vecteur eucaryote selon la revendication 15, caractérisé en ce que l'unité d'expression du gène suicide comprend un gène hybride formé par la fusion d'un gène *tmk* modifié d'*E.coli* et d'un gène *ndk* encodant la nucléoside diphosphokinase d'*E.coli* et codant pour une protéine unique portant à la fois les deux activités thymidylate kinase et thymidine diphosphokinase.

20. Vecteur eucaryote selon la revendication 19, caractérisé en ce que le gène hybride comprend également une fusion avec le gène *ble Sh.*

21. Gène hybride formé par la fusion de deux gènes d'origine bactérienne ou fongique et codant pour une protéine unique ayant à la fois les activités cytosine désaminase et uracile phosphoribosyltransférase.

22. Gène hybride selon la revendication 21, caractérisé en ce que les gènes bactériens sont le gène *codA* codant pour la cytosine désaminase et le gène *upp* codant pour l'uracile phosphoribosyl transférase d'*E.coli.*

23. Gène hybride selon la revendication 21, caractérisé en ce que les gènes fongiques sont le gène *fcyl* codant pour la cytosine désaminase et le gène *furl* codant pour l'uracile phosphoribosyl transférase de *Saccharomyces cerevisiae.*

24. Gène *tmk* d'*E.coli* codant pour la thymidylate kinase fusionné avec un gène conférant une résistance à la zéocine.

25. Gène hybride formé par la fusion du gène *tmk,* selon la revendication 24, et du gène *tdk* encodant la thymidine kinase d'*E.coli* et codant pour une protéine unique ayant les activités thymidine et thymidylate kinase.

26. Gène hybride formé par la fusion du gène *tdk::tmk* selon la revendication 25, et du gène *ble Sh.*

27. Gène hybride formé par la fusion du gène *tmk* selon la revendication 24, et du gène *ndk* encodant la nucléoside diphosphokinase d'*E.coli* et codant pour une protéine unique ayant les activités thymidylate kinase et thymidine diphosphokinase.

28. Gène hybride formé par la fusion du gène *tmk::ndk* selon la revendication 27, et du gène *ble* Sh.

29. Gène *tk* du virus humain Varicella-Zooster encodant les activités de la thymidine et de la thymidylate kinase et fusionné avec un gène portant la résistance à la zéocine.

30. Gène *tk* du virus humain d'Epstein-Barr encodant les activités de la thymidine et de la thymidylate kinase et fusionné avec un gène portant la résistance à la zéocine.

31. Médicament comprenant un vecteur eucaryote selon l'une quelconque des revendications 1 à 20.

32. Médicament pour le traitement des cellules tumorales sensibles à la 5-fluorocytosine et/ou au 5-fluorouracile comprenant un vecteur eucaryote selon l'une quelconque des revendications 1 à 20.

33. Médicament pour le traitement des cellules de mammifères ou des cellules infectées par le VIH comprenant un vecteur eucaryote selon l'une quelconque des revendications 1 à 20.

## Patentansprüche

1. Eukaryotischer Vektor, der zwei Suizidgen-Expressionseinheiten trägt, worin die erste Tumorzellen für 5-Fluorcytosin und/oder 5-Fluoruracil empfindlich macht und die zweite Säugerzellen oder mit HIV infizierte Zellen synergistisch für Azidothymidin sensibilisiert.

2. Eukorytischer Vektor nach Anspruch 1, dadurch gekennzeichnet, daß die erste Suizidgen-Expressionseinheit ein Gen, das eine Uracilphosphoribosyltransferaseaktivität exprimiert, oder ein Hybridgen umfaßt, das aus der Fusion von zwei Genen resultiert, die bakteriellen oder pilzlichen Ursprungs sein können, wobei das Hybridgen für ein einziges Protein codiert, das gleichzeitig die Cytosindesaminase- und die Uracilphosphoribosyltransferaseaktivität besitzt.

3. Eukaryotischer Vektor nach Anspruch 2, dadurch gekennzeichnet, daß die zwei bakteriellen Gene das Gen codA, codierend für die Cytosindesaminase, und das Gen upp, codierend für die Uracilphosphoribosyltransferase von E.coli sind.

4. Eukaryotischer Vektor nach Anspruch 2, dadurch gekennzeichnet, daß die zwei pilzlichen Gene das Gen tcyl, codierend die Cytosindesaminase, und das Gen furl, codierend für die Uracilphosphoribosyltransferase von Saccharomyces cerevisiae sind.

5. Eukaryotischer Vektor nach einem der Ansprüche 1 bis 4, dadurch gekennzeichnet, daß die zweite Suizidgen-Expressionseinheit ein Gen tmk von E.coli codierend für die Thymidylatkinase, fusioniert mit einem Gen, das eine Resistenz gegen Zeocin verleiht, umfaßt.

6. Eukaryotischer Vektor nach einem der Ansprüche 1 bis 4, dadurch gekennzeichnet, daß die zweite Suizidgen-Expressionseinheit ein Hybridgen umfaßt, gebildet durch die Fusion des Gens tmk und eines Gens tdk von E.coli, das für ein einziges Protein codiert und gleichzeitig die Thymidinkinase- und die Thymidylatkinaseaktivität trägt.

7. Eukaroytischer Vektor nach Anspruch 6, dadurch gekennzeichnet, daß das Hybridgen auch eine Fusion mit dem Gen ble Sh umfaßt.

8. Eukaryotischer Vektor nach einem der Ansprüche 1 bis 4, dadurch gekennzeichnet, daß die zweite Suizidgen-Expressionseinheit ein Hybridgen umfaßt, resultierend aus der Fusion eines Gens tmk von E.coli fusioniert mit einem Gen, das eine Resistenz gegen Zeocin verleiht und einem Gen ndk, das für die Nukleosiddiphosphokinase von E.coli codiert, und das für ein einziges Protein codiert, das gleichzeitig die zwei Aktivitäten Thymidylatkinase und Thymidindiphosphokinase trägt.

9. Eukaryotischer Vektor nach Anspruch 8, dadurch gekennzeichnet, daß das Hybridgen auch eine Fusion mit dem Gen ble Sh umfaßt.

10. Eukaryotischer Vektor nach einem der Ansprüche 1 bis 4, dadurch gekennzeichnet, daß die zweite Suizidgen-Expressionseinheit ein modifiziertes Gen tk vom humanen Varicella-Zoster-Virus umfaßt, das für die Thymidinkinase und die Thymydilatkinase codiert, worin das modifizierte Gen die Resistenz gegen Zeocin trägt.

11. Eukaryotischer Vektor nach einem der Ansprüche 1 bis 4, dadurch gekennzeichnet, daß die zweite Suizidgen-Expressionseinheit ein modifiziertes Gen tk vom humanen Epstein-Barr-Virus umfaßt, das für die Thymidinkinase und die Thymidylatkinase codiert, worin das modifizierte Gen die Resistenz gegen Zeocin trägt.

12. Eukaryotischer Vektor, der eine Suizidgen-Expressionseinheit trägt, die die Sensibilisierung von Tumorzellen für 5-Fluorcytosin und/oder 5-Fluoruracil ermöglicht, dadurch gekennzeichnet, daß die Suizidgen-Expressionseinheit ein Gen, das die Uracilphosphoribosyltransferaseaktivität exprimiert oder ein Hybridgen umfaßt, das aus der Fusion von zwei Genen resultiert, die bakteriellen oder pilzlichen Ursprungs sein können, worin das Hybridgen für ein einziges Protein codiert, das gleichzeitig die Cytosindesaminase- und die Uracilphosphoribosyltransferaseaktivität besitzt.

13. Eukaryotischer Vektor nach Anspruch 12, dadurch gekennzeichnet, daß die bakteriellen Gene das Gen codA, codierend für die Cytosindesaminase, und das Gen upp, codierend für die Uracilphosphoribosyltransferase von E.coli sind.

14. Eukaryotischer Vektor nach Anspruch 12, dadurch gekennzeichnet, daß die pilzlichen Gene das Gen fcyl, codierend für die Cytosindesaminase, und das Gen furl, codierend für die Uracilphosphoribosyltransferase von Saccharomyces cerevisiae sind.

15. Eukaryotischer Vektor, der eine Suizidgen-Expressionseinheit trägt, die eine Sensibilisierung für Säugerzellen oder mit dem HIV-Virus infizierten Zellen für Azidothymidin oder andere Analoga von Pyrimidinnukleosiden, wie etwa Didesoxythymidin, Trifluormethylthymidin, Ethyldesoxyuridin, Bromvinyldesoxyuridin, Bromvinylarabinouracil ermöglicht.

16. Eukaryotischer Vektor nach Anspruch 15, dadurch gekennzeichnet, daß die Suizidgen-Expressionseinheit ein Gen tmk von E.coli umfaßt, das für die Thymidylatkinase codiert, fusioniert mit einem Gen, das eine Resistenz gegen Zeocin verleiht.

17. Eukaryotischer Vektor nach Anspruch 15, dadurch gekennzeichnet, daß die Suizidgen-Expressionseinheit ein Hybridgen umfaßt, das aus der Fusion eines modifizierten Gens tmk von E.coli und des Gens tdk von E.coli resultiert, das für die Thymidinkinase codiert, wobei das Hybridgen für ein einziges Protein codiert, das gleichzeitig die Thymidinkinase- und die Thymidylatkinaseaktivität trägt.

18. Eukaryotischer Vektor nach Anspruch 17, dadurch gekennzeichnet, daß das Hybridgen auch eine Fusion mit dem Gen ble Sh umfaßt.

19. Eukaryotischer Vektor nach Anspruch 15, dadurch gekennzeichnet, daß die Suizidgen-Expressionseinheit ein Hybridgen umfaßt, das durch die Fusion eines modifizierten Gens tmk von E. coli und eines Gens ndk gebildet wird, das für die Nukleosiddiphosphokinase von E.coli codiert, und welches für ein einziges Protein codiert, das gleichzeitig die zwei Aktivitäten Thymidylatkinase und Thymidindiphosphokinase trägt.

20. Eukaryotischer Vektor nach Anspruch 19, dadurch gekennzeichnet, daß das Hybridgen auch eine Fusion mit dem Gen ble Sh umfaßt.

21. Hybridgen, das durch die Fusion von zwei Genen bakteriellen oder pilzlichen Ursprungs gebildet ist und das für ein einziges Protein codiert, das gleichzeitig die Cytosindesaminase- und die Uracilphosphoribosyltransferaseaktivität aufweist.

22. Hybridgen nach Anspruch 21, dadurch gekennzeichnet, daß die bakteriellen Gene das Gen codA, das für die Cytosindesaminase codiert, und das Gen upp, das für die Uracilphosphoribosyltransferase von E.coli codiert, sind.

23. Hybridgen nach Anspruch 21, dadurch gekennzeichnet, daß die pilzlichen Gene das Gen fcyl, das für die Cytosindesaminase codiert, und das Gen furl, das für die Uracilphosphoribosyltransferase von Saccharomyces cerevisiae codiert, sind.

24. Gen tmk von E.coli, welches für die Thymidylatkinase codiert, fusioniert mit einem Gen, das eine Resistenz gegen Zeocin verleiht.

25. Hybridgen, das durch die Fusion des Gens tmk gemäß Anspruch 24 und des Gens tdk codierend für die Thymidinkinase von E.coli gebildet ist, und das für ein einziges Protein codiert, das die Thymidin- und die Thymidylatkinaseaktivität aufweist.

26. Hybridgen, das durch die Fusion des Gens tdk::tmk gemäß Anspruch 25 und des Gens ble Sh gebildet ist.

27. Hybridgen, das durch die Fusion des Gens tmk gemäß Anspruch 24 und des Gens ndk, codierend für die Nukleosiddiphosphokinase von E.coli gebildet ist und das für ein einziges Protein codiert, das die Thymidylatkinase- und die Thymidindiphosphokinaseaktivität aufweist.

28. Hybridgen, das durch die Fusion des Gens tmk::ndk gemäß Anspruch 27 und des Gens ble Sh gebildet ist.

29. Gen tk des humanen Varicella-Zoster-Virus, das für die Thymidin- und die Thymidylatkinaseaktivität codiert und das mit einem Gen fusioniert ist, das die Resistenz gegen Zeocin trägt.

30. Gen tk des humanen Epstein-Barr-Virus, das für die Thymidin- und die Thymidylatkinaseaktivität codiert und das mit einem Gen fusioniert ist, das die Resistenz gegen Zeocin trägt.

31. Arzneimittel umfassend einen eukaryotischen Vektor nach einem der Ansprüche 1 bis 20.

32. Arzneimittel zur Behandlung von Tumorzellen, die für 5-Fluorcytosin und/oder 5-Fluoruracil empfindlich sind, umfassend einen eukaryotischen Vektor nach einem der Ansprüche 1 bis 20.

33. Arzneimittel zur Behandlung von Säugerzellen oder von mit HIV infizierten Zellen, umfassend einen eukaryotischen Vektor nach einem der Ansprüche 1 bis 20.

## Claims

1. A eukaryotic vector comprising two expression suicide gene units, the first permitting the sensitization of tumor cells to 5-fluorocytosine and/or 5-fluorouracil and the second permitting the sensitization of mammalian cells or HIV-infected cells to Azidothymidine in a synergistic fashion.

2. The eukaryotic vector according to claim 1, wherein the first expression suicide gene unit comprises a gene expressing uracil phosphoribosyl transferase activity or a hybrid gene, formed by the fusion of two bacterial genes or by the fusion of two fungal genes, said hybrid gene coding for a single protein having both cytosine deaminase and uracil phosphoribosyl transferase activities.

3. The eukaryotic vector according to claim 2, wherein, the two bacterial genes are the *codA* gene encoding cytosine deaminase and the *upp* gene encoding uracil phosphoribosyl transferase from *Escherichia coli.*

4. The eukaryotic vector according to claim 2, wherein the two fungal genes are the *fcy1* gene encoding cytosine deaminase and the *furl* gene encoding uracil phosphoribosyl transferase from *Saccharomyces cerevisiae.*

5. The eukaryotic vector according to anyone of claims 1 to 4, wherein the second expression suicide gene unit comprises a *tmk* gene from *Escherichia coli* encoding thymidylate kinase, fused with a gene conferring zeocin resistance.

6. The eukaryotic vector according to anyone of claims 1 to 4, wherein the second expression suicide gene unit comprises a hybrid gene formed by the fusion of the *tmk* gene and a *tdk* gene from *Escherichia coli*, coding for a single protein with both thymidine kinase and thymidylate kinase activities.

7. The eukaryotic vector according to claim 6, wherein the hybrid gene comprises a further fusion with a *Sh ble* gene.

8. The eukaryotic vector according to anyone of claims 1 to 4, wherein the second expression suicide gene unit comprises a hybrid gene formed by the fusion of a *tmk* gene from *Escherichia coli* fused with a gene conferring zeocin resistance and a ndk gene encoding nucleoside diphosphokinase from *Escherichia coli* and coding for a single protein having both thymidylate kinase and thymidine diphosphokinase activities.

9. The eukaryotic vector according to claim 8, wherein the hybrid gene comprises a further fusion with a *Sh ble* gene.

10. The eukaryotic vector according to anyone of claims 1 to 4, wherein the second expression suicide gene unit comprises a modified *tk* gene from human Varicella-Zoster virus encoding thymidine kinase and thymidylate kinase activities, said modified gene having zeocin resistance.

11. The eukaryotic vector according to anyone of claims 1 to 4, wherein the second expression suicide gene unit comprises a modified *tk* gene from a human Epstein-Barr virus encoding thymidine kinase and thymidylate kinase activities, said modified gene having zeocin resistance.

12. A eukaryotic vector comprising an expression suicide gene unit permitting the sensitization of tumor cells to 5-fluorocytosine and/or 5-fluorouracil, wherein the expression suicide gene unit comprises a gene expressing uracil phosphoribosyl transferase activity or a hybrid gene formed by the fusion of two bacterial genes or by the fusion of two fungal genes, said hybrid gene coding for a single protein having both cytosine deaminase and uracil phosphoribosyl transferase activities.

13. The eukaryotic vector according to claim 12 wherein the bacterial genes are the CodA gene encoding cytosine deaminase and the *upp* gene encoding uracil phosphoribosyl transferase from *Escherichia coli.*

14. The eukaryotic vector according to claim 12, wherein the fungal genes are the *fcy1* gene encoding cytosine deaminase and the fur 1 gene encoding uracil phosphoribosyl transferase from *Saccharomyces cerevisae.*

15. A eukaryotic vector comprising an expression suicide gene unit permitting the sensitization of mammalian cells or HIV-infected cells to Azidothymidine or other pyrimidine nucleoside analogs such as dideoxythymidine, trifluoromethylthymidine, ethyldeoxyuridine, bromovinyldeoxyuridine, bromovinyl-arabinouracil.

16. The eukaryotic vector according to claim 15, wherein the expression suicide gene unit comprises a *tmk* gene from *Escherichia coli* encoding thymidylate kinase fused with a gene conferring zeocin resistance.

17. The eukaryotic vector according to claim 15, wherein the expression suicide gene unit comprises a hybrid gene formed by the fusion of a modified tmk gene from *Escherichia coli,* and the *tdk* gene from *Escherichia coli* coding for thymidine kinase, said hybrid gene coding for a single protein with both thymidine kinase and thymidylate kinase activities.

18. The eukaryotic vector according to claim 17, wherein the hybrid gene comprises a further fusion with a *Sh ble* gene.

19. The eukaryotic vector according to claim 15, wherein the expression suicide gene unit comprises a hybrid gene formed by the fusion of a modified *tmk* gene from *Escherichia coli* and a *ndk* encoding nucleoside diphosphokinase from *Escherichia coli* and coding for a single protein having both thymidylate kinase and thymidine diphosphokinase activities.

20. The eukaryotic vector according to claim 19, wherein the hybrid gene comprises a further fusion with a *Sh ble* gene.

21. A hybrid gene formed by the fusion of two bacterial or by the fusion of two fungal genes and coding for a single protein with both cytosine deaminase and uracil phosphoribosyl transferase activities.

22. The hybrid gene according to claim 21 wherein the bacterial genes are the *codA* gene encoding cytosine deaminase and the *upp* gene encoding uracil phosphoribosyl transferase from *Escherichia coli.*

23. The hybrid gene according to claim 21 wherein the fungal genes are the *fcy1* gene encoding cytosine deaminase and the *fur1* gene encoding uracil phosphoribosyl transferase from *Saccharomyces cerevisiae.*

24. A *tmk* gene from *Escherichia coli* encoding thymidylate kinase, fused with a gene conferring zeocin resistance.

25. A hybrid gene formed by the fusion of the tmk gene according to claim 24 and the *tdk* encoding thymidine kinase gene from *Escherichia coli* and coding for a single protein with both thymidine and thymidylate kinase activities.

26. A hybrid gene formed by the fusion of the *tdk::tmk* gene according to claim 25 and the *Sh ble* gene.

27. A hybrid gene formed by the fusion of the *tmk* gene according to claim 24 and the ndk encoding nucleoside diphosphokinase from *Escherichia coli* and coding for a single protein with both thymidylate kinase and thymidine diphosphokinase activities .

28. A hybrid gene formed by the fusion of the *tmk::ndk* gene according to claim 27 and the *Sh ble* gene.

29. A *tk* gene from a human varicella-zoster virus encoding thymidine and thymidylate kinase activities and fused with a gene conferring Zeocin resistance.

30. A *tk* gene from a human Epstein-Barr virus encoding thymidine and thymidylate kinase activities and fused with a gene conferring Zeocin resistance.

31. Medicament comprising a encaryotic vector according to one of claims 1 to 20.

32. Medicament for the treatment of tumor cells sensitive to 5-fluorocytosine and/or 5-fluorouracil, comprising a eucaryotic vector according to one of claims 1 to 20.

33. Medicament for the treatment of mammalian cells or cells infected by HIV, comprising a eucaryotic vector according to one of claims 1 to 20.
